# EUROPEAN PATENT SPECIFICATION

(11) **EP 3 411 040 B1**
(45) Date of publication and mention of the grant of the patent: **11.11.2026**
(21) Application number: 17748101.7
(22) Date of filing: 01.02.2017
(51) Int. Cl.: A61K 31/553, A61K 31/56, A61K 31/58, A61K 31/606, A61K 31/7076, A61K 39/395, A61P 21/00

(54) **SMALL MOLECULES FOR MOUSE SATELLITE CELL PROLIFERATION**
KLEINE MOLEKÜLE FÜR MAUSSATELLITENZELLPROLIFERATION
PETITES MOLÉCULES POUR LA PROLIFÉRATION DE CELLULES SATELLITES DE SOURIS

(30) Priority: 01.02.2016 US 201615012656
(43) Date of publication of application: 12.12.2018
(73) Proprietor: President and Fellows of Harvard College, Cambridge, MA 02138 (US)
(72) Inventor: RUBIN, Lee, L., Wellesley MA 02481 (US); WAGERS, Amy J., Cambridge Massachusetts 02138 (US); GEE, Amanda K. W., Alexandria VA 22314 (US); PRICE, Feodor, D., Somerville MA 02145 (US)
(74) Representative: Lock, Graham James
(86) International application number: PCT/US2017/016099
(87) International publication number: WO 2017/136480

(56) References cited:
- WO-A2-2011/109834
- WO-A2-2012/030919
- US-A1- 2003 181 510
- US-A1- 2007 224 168
- US-A1- 2009 306 087
- US-A1- 2014 294 855
- COSGROVE ET AL.: "Rejuvenation of the Muscle Stem Cell Population Restores Strength to Injured Aged Muscles", NATURE MEDICINE, vol. 20, no. 3, 3 January 2014 (2014-01-03), pages 255 - 264, XP055405393

## Description

### TECHNICAL FIELD

The present disclosure relates generally to compositions and methods of promoting satellite cell proliferation.

### BACKGROUND

Satellite cells are a population of skeletal muscle stem cells that are located beneath the basal lamina surrounding the muscle fiber and are required for muscle growth and muscle repair after injury or exercise. Satellite cell number and function are affected by normal aging and in several diseases, resulting in progressive muscle wasting or inefficient recovery after injury. Examples include Duchenne muscular dystrophy, in which satellite cells are depleted through constant use, and sarcopenia, where satellite cells may both be depleted and adversely affected in their proliferative capacity by changes in their environment (Jejurikar and Kuzon, Apoptosis, 8 (2003), 573-578). Finding treatments that would expand the endogenous population of satellite cells could aid greatly in treating these debilitating diseases.

US 2014/294855 A1 describes kinase inhibitors, among which are B-Raf inhibitors, for use in increasing satellite cell proliferaton.

There is need in the art for compositions and methods for inducing satellite cell proliferation.

### SUMMARY

The inventors conducted an image-based screen of selected small molecules for their ability to increase proliferation in satellite cells isolated from adult mouse muscle tissue. Satellite cells were isolated using cell surface markers (Sherwood et al., Cell, 119 (2004), 543-554), cultured in the presence of small molecules for four days, and then analyzed using automated confocal microscopy to determine cell number. Using this procedure, the inventors discovered several compounds, operating through defined signaling pathways, which can enhance proliferation in cultures of primary mouse satellite cells. The myogenic capacity of treated cells is currently being tested through marker analysis and differentiation assays, as well as in vivo muscle grafting. The compounds are capable of proliferating cells without adversely affecting their differentiation potential and can be used for treatment of diseases having a skeletal muscle defect as one of their components. These compounds are also referred to as proliferation enhancers herein.

The invention is set out in the appended set of claims. Accordingly, the invention is a method of increasing satellite cell proliferation, the method comprising: contacting a satellite cell *in vitro* with a B-Raf inhibitor selected from the group consisting of BAY-439006 (i.e., Sorafenib; HMSL10008-101-1); HG-6-64-01 (i.e., HMSL10017-101-1); SB590885 (i.e., GSK2118436; HMSL10046-101-1); AZ-628 (i.e., HMSL10050-101-1); GDC-0879 (i.e., HMSL10181-101-1); RAF 265 (i.e., HMSL10206-101); TAK-632 (i.e., HMSL10409-101); and any combinations thereof.

In another aspect, provided herein is a B-Raf inhibitor selected from the group consisting of BAY-439006 (i.e., Sorafenib; HMSL10008-101-1); HG-6-64-01 (i.e., HMSL10017-101-1); SB590885 (i.e., GSK2118436; HMSL10046-101-1); AZ-628 (i.e., HMSL10050-101-1); GDC-0879 (i.e., HMSL10181-101-1); RAF 265 (i.e., HMSL10206-101); TAK-632 (i.e., HMSL10409-101); and any combinations thereof for use in muscle repair or regeneration in a subject which has a damaged muscle tissue.

### BRIEF DESCRIPTION OF THE DRAWINGS

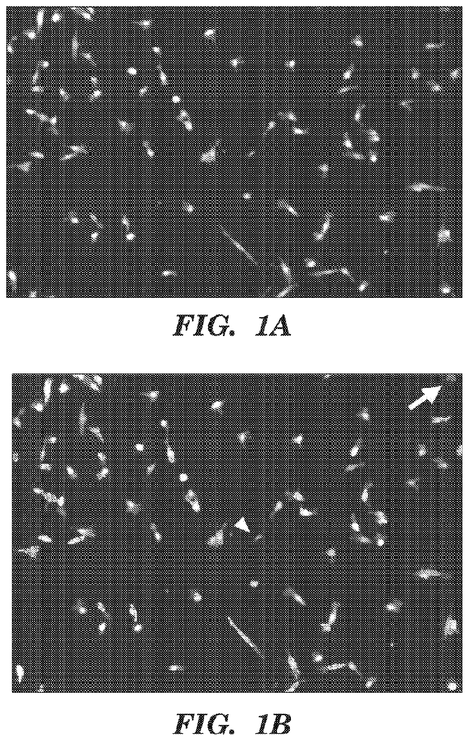

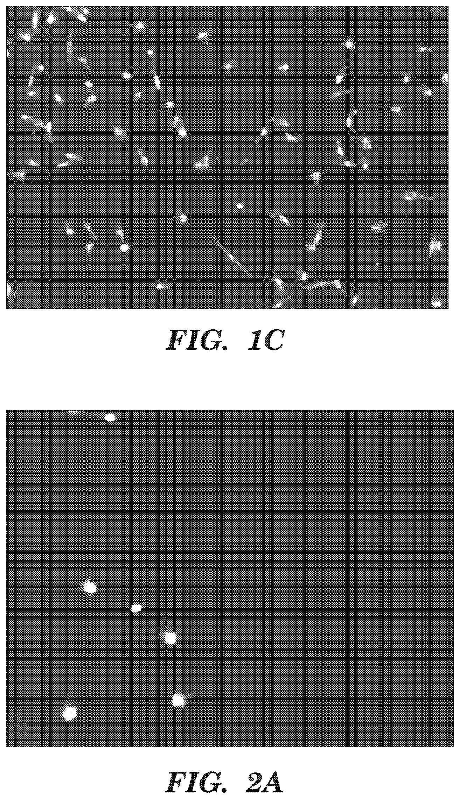

**FIGS. 1A-1C** show Opera analysis of satellite cell number. Images were captured using the Perkin Elmer Opera automated confocal imaging system. Since all cells isolated from the CAG-EGFP animals were fluorescent, the inventors could image the cells directly (**FIG. 1A**). Images were analyzed using Acapella software to count cells that had signals within the set thresholds for each parameter (**FIG. 1B**). Cells or debris that were too dim (arrow), or too small (arrowheads) were excluded. Cells and debris that were highly fluorescent or too large in size were also excluded. Cells could then be further grouped according to set criteria, such as roundness (**FIG. 1C**).
**FIGS. 2A-2D** show analysis of positive hit compounds. Adult mouse satellite cells were isolated via FACS, based on the procedure outlined in Sherwood et al. (Cell, 119 (2004), 543-554). Then, cells were treated with compounds and allowed to proliferate for four days, fixed, and imaged. Proliferation induced by compounds was compared to that found with a DMSO vehicle control (**FIG. 2A**), or to that found with bFGF (**FIG. 2B**). Two example images from positive hit reference compounds are shown, Flt3 Kinase inhibitor (**FIG. 2C**) and Adenosine receptor agonist (**FIG. 2D**).

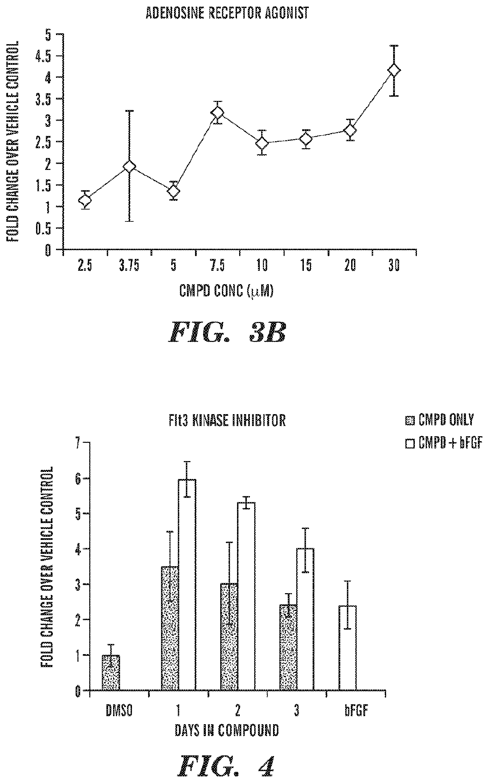

**FIGS. 3A** and **3B** show validation of some exemplary hit reference compounds. Compounds identified in the primary screen as hits were then tested in a dose response assay. The level of proliferation is measured as fold change over the DMSO vehicle control value of (A.1±0.3 (**FIG. 3A**) and 1±0.4 (**FIG. 3B**). For comparison, the bFGF positive control values were 3.4±0.9 (**FIG. 3A**) and 5.6±1.7 (**FIG. 3B**).
**FIG. 4** shows optimization of culture conditions. Culture conditions were optimized by testing the effects of compounds with differential exposure time, with or without the presence of bFGF. For all time points, media were replaced with standard proliferation medium on the indicated days. For this reference compound, the inclusion of bFGF had an additive effect on proliferation. Also, while the compound itself led to proliferation similar to the bFGF positive control at all time points, it was seen to be more effective with a shorter exposure time.

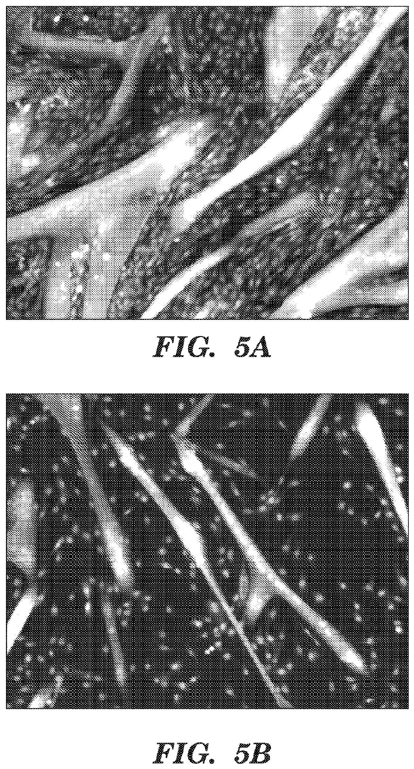

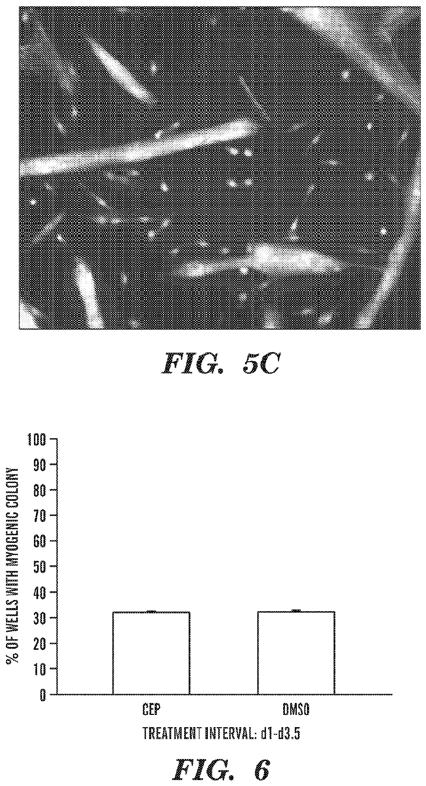

**FIGS. 5A-5C** show differentiation of treated cultures. CAG-EGFP satellite cells were grown in the inventors' standard proliferation conditions and exposed to compounds for four days. Under the inventors' standard proliferation conditions cells were cultured on laminin-coated plates in Ham's F-10 medium supplemented with 10% heat inactivated horse serum, 100Units/mL penicillin/ 100ug/mL streptomycin, and 2mM L-glutamine. Compounds were added the day after plating. The compounds and media were either refreshed daily or after three days. To differentiate cells and form myotubes, after four days in proliferation conditions, media was switched to high-glucose DMEM supplemented with 10% heat inactivated horse serum, 10% fetal bovine serum, 0.5% chick embryo extract, 100Units/mL penicillin/ 100ug/mL streptomycin, and 2mM L-glutamine. Cultures were grown in differentiation media for 3-5 days until myotube formation was observed, then fixed. Cultures were then switched into differentiation media and cultured another four days. They were then fixed and stained with anti-myosin heavy chain (red channel) and Hoechst (blue channel). Cells grown with only DMSO vehicle control did not form myotubes because they were not dense enough in culture (data not shown). Cells grown in the presence of bFGF positive control (**FIG. 5A**) were able to form large myotubes. Cultures grown in the presence of a reference Flt3 Kinase inhibitor (**FIG. 5B**) and a reference adenosine agonist (**FIG. 5C**) were able to form myotubes as well.
**FIG. 6** shows myogenic colony formation of single SMPs treated with CEP/DMSO for 5 days. Treatment interval d1-d3.5 and n=3.

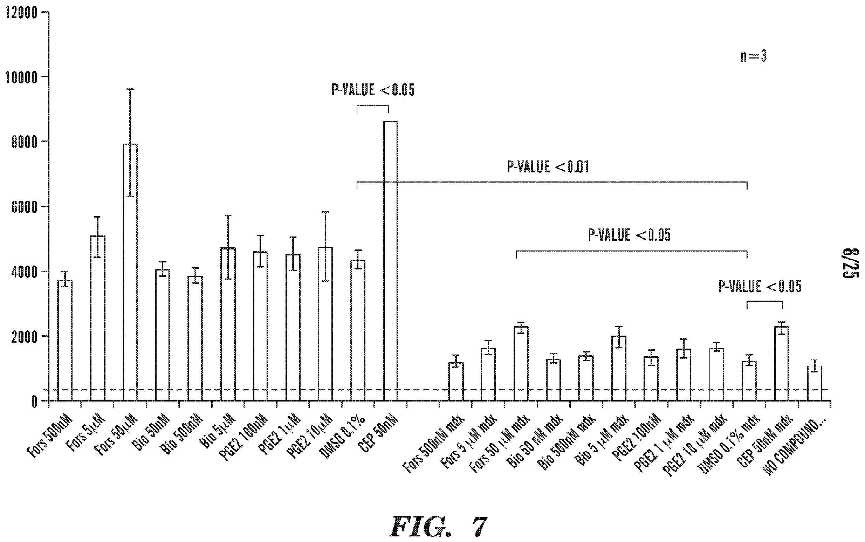

**FIG. 7** shows Exposure to compound from d2 to d4.5. Initial cell number was 250 and n= 3.

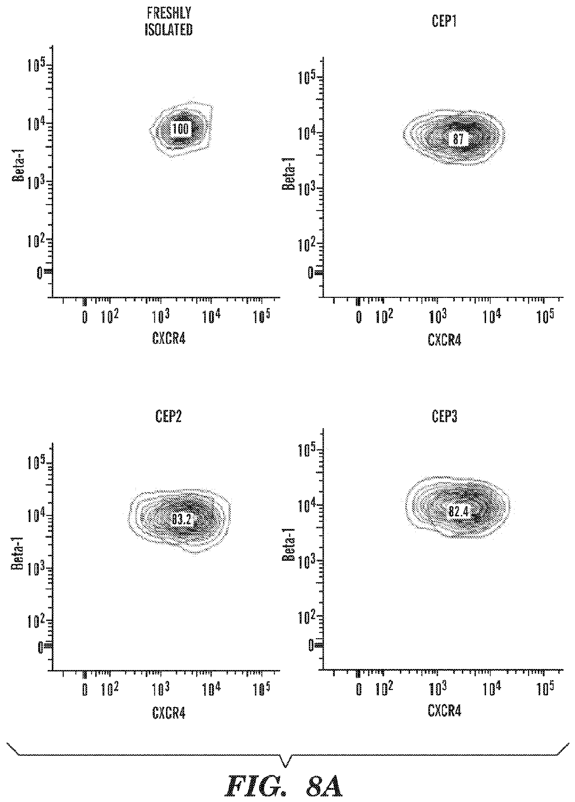

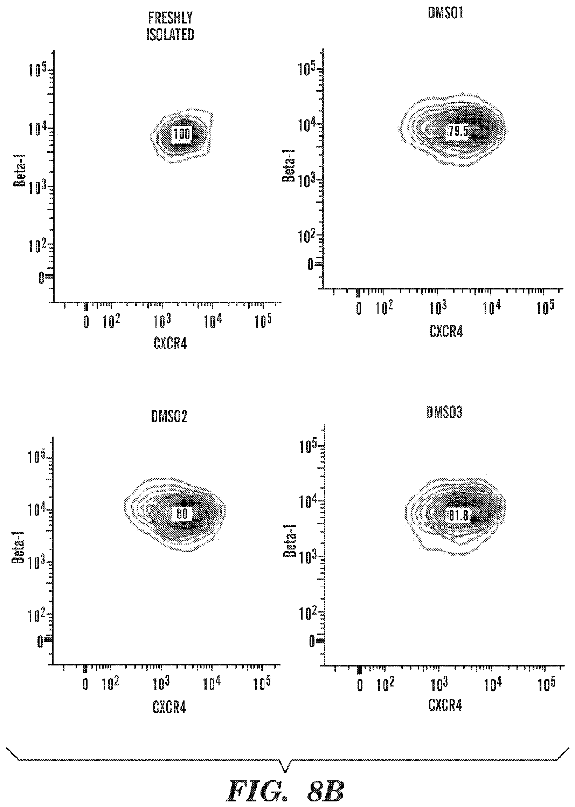

**FIGS. 8A** and **8B** show expression of CXCR4 and Beta-1 integrin on cultured SMPs after 5 days: CEP (**FIG. 8A**) and DMSO (**FIG. 8B**).
**FIGS. 9-43****,** **45****,** **50** show results for reference compounds.
**FIGS. 9A-13** show dose response curves for some exemplary hit compounds. Shown are Sunitinib (SU11248, **FIGS. 9A** and **9B**), Jak3 inhibitor VI (**FIGS. 10A** and **10B**), Lestaurtinib (CEP701, **FIGS. 11A** and **11B**), Bosutinib (**FIG. 12**), and SU11652 (**FIG. 13**).
**FIGS. 14 -17** are bar graphs showing the synergestic effect of bFGF with CEP701 (**FIGS. 14 and 15**) and Jak3 inhibitor VI (**FIG. 16**), and Sunitinib (**FIG. 17**) on proliferation of satellite cells.
**FIG. 18-20** are photographs showing differentiation of satellite cells after treatment with CEP701 (**FIG. 18**), Sunitinib (**FIG. 19**), and Jak3 inhibitor VI (**FIG. 20**).
**FIG. 21** is abar graph showing the synergistic effect of CEP701 with a TGF-beta inhibitor (Alk5 inhibitor II).
**FIG. 22** shows specificity of CEP701 for proliferating satellite cells. Left panel: CEP701; right panel: DMSO.
**FIG. 23** is bar graph showing CEP701 has no effect on primary fibroblasts
**FIGS. 24** and **25** are bar graphs showing CEP701 is effective in both the aged (**FIG. 24**) and young (**FIG. 25**) tissue.
**FIGS. 26** and **27** are line graph showing dose response curves for N6-cyclopentyladenosine (**FIG. 26**) and Budesonide (**FIG. 27**).
**FIGS. 28** and **29** are bar graph showing the synergistic effect of bFGF with N6-cyclopentyladenosine (**FIG. 28**) and Budesonide (**FIG. 29**).
**FIGS. 30** and **31** are photographs showing differentiation of satellite cells after treatment with N6-Cyclopentyladenosine (**FIG. 30**) and Budesonide (**FIG. 31**).
**FIG. 32** is a bar graph showing the synergestic effect of N6-Cyclopentyladenosine with a TGF-beta inhibitor (Alk5 inhibitor II).
**FIG. 33** illustrates the screening assay performed to identify primary and secondary compounds that were shown to increase satellite cell proliferation *in vitro.*
**FIG. 34** depicts the customer screening library used by the present inventors to screen a set of approximately 400 compounds to identify compounds that increase satellite cell proliferation.
**FIG. 35** illustrates the results of assays performed and that demonstrate that lestaurtinib (CEP701), Sunitinib (SU11248), JAK3 inhibitor VI, and N6-cyclopentyladenosine (CPA) were found to increase *in vitro* satellite cell proliferation. Lestaurtinib (CEP701) was identified as a top hit, was effective at nanomolar doses and had target overlap with several other hit compounds.
**FIG. 36** illustrates the results of an assay performed and that evidence that lestaurtinib (CEP701) increased proliferation of aged satellite cells *in vitro.*
**FIGS. 37A-37B** illustrate a dose response assay (**FIG. 37A**) and the response curves for each of lestaurtinib (CEP701), sunitinib (SU11248), JAK3 inhibitor VI, and N6-cyclopentyladenosine (CPA) (**FIG. 37B**).
**FIG. 38** demonstrates that both CEP-701 and AC220 increased human satellite cells by more than 2-fold at a concentration of 1nM relative to control (DMSO).
**FIG. 39** presents the results of an assay performed and confirms that compounds identified as hits (e.g., CEP701, SU11248, JAK3 inhibitor VI, CPA and Tyr AG490) drive myoblast differentiation.
**FIG. 40** demonstrates that CEP701 enhances myoblast differentiation in differentiation media relative to the DMSO control, as evidenced by the observed increase in both myoblast area and length.
**FIGS. 41A-41B** depict an experimental protocol (**FIG. 41A**) and the results of that experiment (**FIG. 41B**) and which illustrates that CEP701 treatment of cells resulted in an increased number of GFP+ fibers per section, relative to control (DMSO).
**FIGS. 42A-42D** depict an experimental protocol (**FIG. 42A**) and the corresponding results observed. As illustrated in **FIGS. 42B-42D****,** treatment with CEP701 increased both regenerating fiber size and satellite cell number *in vivo* in both adult and aged mice.
**FIGS. 43A-43B** illustrate the affect that the identified compounds have on RTKs (**FIG. 43A**) and, as illustrated in **FIG. 43B****,** compares the fold change in phospho-RET in both uninjured contralateral tibialis anterior (TA) muscle to that observed 2 days post-cardiotoxin injury. As illustrated in **FIG. 43B****,** 2 days post-cardiotoxin injury, an approximately 8-fold increase in phospho-RET was observed by ELISA relative to uninjured contralateral TA muscle.
**FIGS. 44A-44B** illustrate that satellite cells express RET *in vitro.*
**FIGS. 45A-45D** illustrate that CEP701 treatment inhibits RET phosphorylation *in vitro.*
**FIG. 46** shows the results of a study evaluating the effects of the *in vitro* deletion of RET.
**FIG. 47** illustrates that by using a conditional RET mutant and reporter, the present inventors were able to determine that the RET promoter is active in at least 25% of satellite cells.
**FIG. 48** shows that that RET knockout cells proliferate better than wild-type cells *in vitro* (n=6; p=0.0004).
**FIG. 49** demonstrates the fold change relative to control of untreated FLT3 and RET knockout cells.

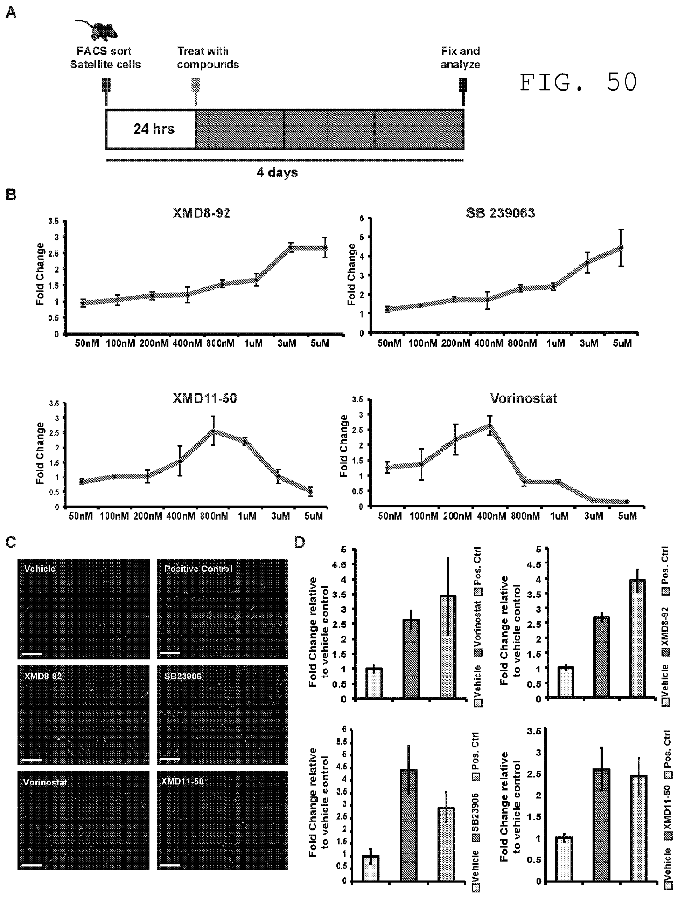

**FIG. 50** **identifies small molecules that promote satellite cell proliferation.** (A) Chemical screen experimental schematic outlining FACS isolation and compound library treatment of satellite cells. (B) Representative dose response curves from four of the top ten compounds. Top ten compounds were chosen based on highest fold change of cell proliferation relative to vehicle controls. Proliferation was assessed via high content imaging using Hoechst 33342 as a cell marker. (C) Representative fluorescent images of FACS sorted satellite cells from Tg:Pax7-nGFP mice on 96w plates cultured for 4 days and treated with vehicle, compound or positive control (Jak3 inhibitor 6). Optimal treatment concentration for each compound was determined in dose response; 3uM for XMD8-92, 5uM for SB23906, 800nM for XMD11-50, and 400nM for Vorinostat. Hoechst 33342 was used as a cell marker. Scale bars denote 100um. (D) Fold change relative to vehicle control for several compounds that promote satellite cell expansion.

### DETAILED DESCRIPTION OF EXEMPLARY EMBODIMENTS

Provided herein is a method of increasing satellite cell proliferation. The method comprising contacting a satellite cell *in vitro* with a B-Raf inhibitor selected from the group consisting of BAY-439006 (i.e., Sorafenib; HMSL10008-101-1); HG-6-64-01 (i.e., HMSL10017-101-1); SB590885 (i.e., GSK2118436; HMSL10046-101-1); AZ-628 (i.e., HMSL10050-101-1); GDC-0879 (i.e., HMSL10181-101-1); RAF 265 (i.e., HMSL10206-101); TAK-632 (i.e., HMSL10409-101); and any combinations thereof.

As used herein, the term "proliferation" means growth and division of cells. In some embodiments, the term "proliferation" as used herein in reference to cells refers to a group of cells that can increase in number over a period of time.

As used herein, "inducing,", "enhancing," or "increasing" satellite cell proliferation means that satellite cells replicate at a faster rate and/or more frequently. In some embodiments of this and other aspects described herein, satellite cell proliferation is increased by at least 5%, 10%, 20%, 30%, 40%, 50%, 50%, 70%, 80%, 90%, 1-fold, 1.1-fold, 1.5-fold, 2-fold, 3-fold, 4-fold, 5-fold, 10-fold, 50-fold, 100-fold or more higher relative to an untreated control. The % or fold increase in satellite cell proliferation can be determined by measuring number of replicating satellite cells while in contact with a compound described herein relative to a control where the satellite cells are not in contact with the compound. Increase in proliferation can also be based on ratios of replicating cells to total number of cells in the respective treated and untreated control. In some embodiments, total number of cells in the treated and untreated controls is used to determine the proliferation. Satellite cell proliferation can be determined using the BrdU incorporation method described in U.S. Patent Publication No. 2009/0136481.

Myosatellite cells or satellite cells are small mononuclear progenitor cells with virtually no cytoplasm found in mature muscle. They are found sandwiched between the basement membrane and sarcolemma (cell membrane) of individual muscle fibers, and can be difficult to distinguish from the sub-sarcolemmal nuclei of the fibers. Satellite cells are able to differentiate and fuse to augment existing muscle fibers and to form new fibers. These cells represent the oldest known adult stem cell niche, and are involved in the normal growth of muscle, as well as regeneration following injury or disease.

In undamaged muscle, the majority of satellite cells are quiescent; they neither differentiate nor undergo cell division. In response to mechanical strain, satellite cells become activated. Activated satellite cells initially proliferate as skeletal myoblasts before undergoing myogenic differentiation.

Markers characteristic of satellite cells include the expression of cell surface proteins or the encoding genes, the expression of intracellular proteins or the encoding genes, cell morphological characteristics, and the like. Those skilled in the art will recognize that known immunofluorescent, immunochemical, polymerase chain reaction, in situ hybridization, Northern blot analysis, chemical or radiochemical or biological methods can readily ascertain the presence or absence of satellite cell specific characteristics.

If desired, the type(s) of cells in a population of satellite can be determined using techniques that are well known in the art. For example, the use of cell-type specific stains. Alternatively, one can perform immunofluorescence staining using antibodies directed to various satellite cell specific proteins. In addition, a cell type can be determined by its morphology using techniques such as, for example, light microscopy, or electron microscopy.

Satellite cells express a number of distinctive genetic markers. For example, current thinking is that all satellite cells express PAX7 and PAX3 (F. Rlaix et al. Nature, 2005, 435(7044): 898-899). Activated satellite cells express myogenic transcription factors, such as Myf5 and MyoD. They also begin expressing muscle-specific filament proteins such as desmin as they differentiate.

Little is known of the regulation of satellite cells. Whilst together PAX3 and PAX7 currently form the definitive satellite markers, Pax genes can be poor transcriptional activators. The dynamics of activation and quiesence and the induction of the myogenic program through the myogenic regulatory factors, Myf5, MyoD, myogenin, and MRF4 remains to be determined. There is some research indicating that satellite cells are negatively regulated by a protein called myostatin. Increased levels of myostatin up-regulate a cyclin-dependent kinase inhibitor called p21 and thereby induce the differentiation of satellite cells.

In some embodiments, the satellite cells are in a stabilized state, e.g., the cells were taken from a subject and treated in such a manner as to allow them to be stored for some period of time. For example, the cells can be frozen, e.g., using methods known in the art for freezing primary cells, such that the cells are viable when thawed. For example, methods known in the art to freeze and thaw embryos to generate live mammals can be adapted for use in the present methods. Such methods can include the use of liquid nitrogen, e.g., with one or more cryoprotectants, e.g., agents that prevent freeze-thaw damage to the cell.

### Kinase inhibitors

As used herein, the term "kinase" means any phosphotransferase enzyme that transfers a phosphate group. In some embodiments, the kinase is a protein kinase. Protein kinases are a family of enzymes that catalyse the phosphorylation of specific residues in proteins. In general protein kinases fall into several groups; those which preferentially phosphorylate serine and/or threonine residues, those which preferentially phosphorylate tyrosine residues and those which phosphorylate both tyrosine and Ser/Thr residues.

Protein kinases include, for example, but are not limited to, members of the Protein Tyrosine Kinase family (PTKs), which in turn can be divided into the cytoplasmic PTKs and the receptor PTKs (RTKs). The cytoplasmic PTKS include the SRC family, (including: BLK; FOR; FYN; HCK; LCK; LYN; SRC; YES and YRK); the BRK Family (including: BRK; FRK, SAD; and SRM); the CSK family (including: CSK and CTK); the BTK family, (including: BTK; ITK; TEC; MKK2 and TXK), the Janus kinase family, (including: JAKI, JAK2, JAK3 and Tyk2), the FAK family (including, FAK and PYK2); the Fes family (including FES and FER), the ZAP70 family (including ZAP70 and SYK); the ACK family (including ACK1 and ACK2); and the Abl family (including ABL and ARG). The RTK family includes the EGF-Receptor family (including, EGFR, HER2, HER3 and HER4); the Insulin Receptor family (including INS-R and IGF1-R); the PDGF-Receptor family (including PDGFRa, PDGFR|3, CSF1R, KIT, FLK2); the VEGF-Receptor family (including; FLT1, FLK1 and FLT4); the FGF-Receptor family (including FGFR1, FGFR2, FGFR3 and FGFR4); the CCK4 family (including CCK4); the MET family (including MET and RON); the TRK family (including TRKA, TRKB, and TRKC); the AXL family (including AXL, MER, and SKY); the TIE/TEK family (including TIE and TIE2/TEK); the EPH family (including EPHA1, EPHA2, EPHA3, EPHA4, EPHA5, EPHA6, EPHA7, EPHA8, EPHB1, EPHB2, EPHB3, EPHB4, EPHB5, EPHB6); the RYK family (including RYK); the MCK family (including MCK and TYRO 10); the ROS family (including ROS); the RET family (including RET); the LTK family (including LTK and ALK); the ROR family (including ROR1 and ROR2); The Musk family (including Musk); the LMR family including LMR1, LMR2 and LMR3); and the SuRTK106 family (including SuRTK106).

Representative, non-limiting examples of kinases include Abl, Abl(T315I), ALK, ALK4, AMPK, Arg, Arg, ARKS, ASK1, Aurora-A, Axl, Blk, Bmx, BRK, BrSKl, BrSK2, BTK, CaMKI, CaMKII, CaMKIV, CDKl/cyclinB, CDK2/cyclinA, CDK2/cyclinE, CDK3/cyclinE, CDK5/p25, CDK5/p35, CDK6/cyclinD3, CDK7/cyclinH/MATl, CDK9/cyclin Tl, CHK1, CHK2, CKl(y), CK18, CK2, CK2a2, cKit(D816V), cKit, c-RAF, CSK, cSRC, DAPK1, DAPK2, DDR2, DMPK, DRAK1, DYRK2, EGFR, EGFR(L858R), EGFR(L861Q), EphAl, EphA2, EphA3, EphA4, EphAS, EphAV, EphAS, EphBl, EphB2, EphB3, EphB4, ErbB4, Per, Fes, FGFR1, FGFR2, FGFR3, FGFR4, Fgr, Fill, Flt3(D835Y), Flt3, Flt4, Fms, Fyn, GSK3(3, GSK3a, Hck, HIPK1, HIPK2, HIPK3, IGF-1R, IKK(3, IKKa, IR, IRAKI, IRAK4, IRR, ITK, JAK2, JAK3, JNKlal, JNK2a2, JNK3, KDR, Lck, LIMK1, LKB1, LOK, Lyn, Lyn, MAPK1, MAPK2, MAPK2, MAPKAP-K2, MAPKAP-K3, MARK1, MEK1, MELK, Met, MINK, MKK4, MKK6, MKK7(3, MLCK, MLK1, Mnk2, MRCK-beta, MRCKa, MSK1, MSK2, MSSK1, MST1, MST2, MST3, MuSK, NEK2, NEKS, NEK6, NEK7, NLK, p70S6K, PAK2, PAK3, PAK4, PAK6, PAR-lBa, PDGFR(3, PDGFRa, PDK1, PI3K beta, PI3K delta, PI3K gamma, Pim-1, Pim-2, PKA(b), PKA, PKB(3, PKBa, PKBy, PKC^i, PKC(3I, PKC(3II, PKCa, PKCy, PKC8, PKCe, PKC^, PKCrl, PKC9, PKCi, PKD2, PKG1(3, PKGla, Plk3, PRAK, PRK2, PrKX, PTK5, Pyk2, Ret, RIPK2, ROCK-I, ROCKII, ROCK-II, Ron, Ros, Rse, Rskl, Rskl, Rsk2, Rsk3, SAPK2a, SAPK2a(T106M), SAPK2b, SAPK3, SAPK4, SGK, SGK2, SGK3, SIK, Snk, SRPK1, SRPK2, STK33, Syk, TAK1, TBK1, Tie2, TrkA, TrkB, TSSK1, TSSK2, WNK2, WNK3, Yes, ZAP-70, ZIPK.

As used herein, the term "kinase inhibitor" means any compound, molecule or composition that inhibits or reduces the activity, e.g., phosphotransferase activity, of a kinase. Without limitations, a kinase inhibitor can be selected from the group consisting of small or large organic or inorganic molecules; monosaccharides; disaccharides; trisaccharides; oligosaccharides; polysaccharides; biological macromolecules, e.g., proteins, peptides, peptide analogs and derivatives thereof, peptidomimetics, nucleic acids, nucleic acid analogs and derivatives, enzymes, antibodies, portion or fragments of antibodies; an extract made from biological materials such as bacteria, plants, fungi, or animal cells or tissues; naturally occurring or synthetic compositions; and any combinations thereof.

The kinase inhibitor according to the invention is a B-Raf inhibitor selected from the group consisting of is BAY-439006 (i.e., Sorafenib; HMSL10008-101-1); HG-6-64-01 (i.e., HMSL10017-101-1); SB590885 (i.e., GSK2118436; HMSL10046-101-1); AZ-628 (i.e., HMSL10050-101-1); GDC-0879 (i.e., HMSL10181-101-1); RAF 265 (i.e., HMSL10206-101); TAK-632 (i.e., HMSL10409-101); and any combinations thereof.

In some embodiments of this and other aspects described herein, activity of the kinase is inhibited or lowered by at least 5%, at least 10%, at least 20%, at least 30%, at least 40%, at least 50%, at least 60%, at least 70%, at least 80%, at least 90%, at least 95%, at least 98%, or 100% (e.g. complete loss of activity) relative to an uninhibited control. Without wishing to be bound by theory, activity of a kinase can be determined using any assay known in the art for measuring the activity of the kinase, e.g., by measuring phosphorylation reactions.

### Contacting of satellite cells with compounds

The satellite cell population can be contacted with the proliferation enhancer in a cell culture e.g., *in vitro* or *ex vivo.* The proliferation enhancer described herein is for use in repairing or regenerating a damaged muscle tissue in a subject.

The term "contacting" or "contact" as used herein in connection with contacting a population of satellite cells includes subjecting the satellite cells to an appropriate culture media which comprises the indicated compound or agent.

Methods of administering compounds to a subject are known in the art and easily available to one of skill in the art. Promoting satellite cell proliferation in a subject can lead to treatment, prevention or amelioration of a number of diseases, disorders or conditions which are caused by a damaged muscle tissue.

Satellite cells suitable for use in *ex vivo* methods can be obtained from subject according to methods well known to those skilled in the art.

The term "*ex vivo*" refers to cells which are removed from a living organism and cultured outside the organism (e.g., in a test tube). For *ex vivo* methods, satellite cells can include autologous satellite cells, i.e., a cell or cells taken from a subject who is in need of treatment for muscle damage or repair. Autologus satellite cells have the advantage of avoiding any immunologically-based rejection of the cells. Alternatively, the cells can be heterologous, e.g., taken from a donor. The second subject can be of the same or different species. Typically, when the cells come from a donor, they will be from a donor who is sufficiently immunologically compatible with the recipient, i.e., will not be subject to transplant rejection, to lessen or remove the need for immunosuppression. In some embodiments, the cells are taken from a xenogeneic source, i.e., a non-human mammal that has been genetically engineered to be sufficiently immunologically compatible with the recipient, or the recipient's species. Methods for determining immunological compatibility are known in the art, and include tissue typing to assess donor-recipient compatibility for HLA and ABO determinants. See, e.g., Transplantation Immunology, Bach and Auchincloss, Eds. (Wiley, John & Sons, Incorporated 1994).

Without wishing to be bound by theory any suitable cell culture media can be used for *ex vivo* methods of the invention. For example, inventors have disovered that the cells can survive in minimum of 10% serum medium. While the cells can survive in suspension culture with at elast 30% serum, they may need to be adherent when medium with 10% serum is used in absence of bFGF. Cells are optimal in cultures when they are adherent on laminin. After *ex vivo* contact with a compound described herein, when the satellite cells have reached a desired population number or density, e.g., about 1x10⁶, 2x10⁶, 3x10⁶, 4x10⁶, 5x10⁶, 6x10⁶, 7x10⁶, 8x10⁶, 9x10⁶, 1x10⁷, 2x10⁷, or more cells, the cells can be transplanted in a subject who is in need of treatment for muscle repair or damage. The cells can be transplanted in a subject from whom the cells were originally obtained or in different subject.

When the satellite cells are contacted with a proliferation enhancer, the proliferation enhancer can have a direct or an indirect affect on the satellite cells. As used herein, a "direct affect" means that the proliferation enhancer is directly interacting with the satellite cells, e.g., binding to a cell surface receptor on the satellite cell, taken up into the satellite cells. As used herein, an "indirect affect" means that the proliferation enhancer does not directly interacts with the satellite cell. For example, the proliferation enhancer can interact with a non-satellite cell and indirectly influence the proliferation of satellite cell. Without wishing to be bound by theory, the proliferation enhancer can indirectly influence a satellite cell by inducing expression and/or secretion of a molecule from a non-satellite cell, and this molecule then directly or indirectly influencing the proliferation of satellite cells.

As used herein, the term "damaged muscle tissue" refers to a muscle tissue, such as a skeletal or cardiac muscle that has been altered for instance by a physical injury or accident, disease, infection, over-use, loss of blood circulation, or by genetic or environmental factors. A damaged muscle tissue can be a dystrophic muscle or an ageing muscle. Exemplary symptoms of muscle damage include, but are not limited to, swelling, bruising or redness, open cuts as a consequence of an injury, pain at rest, pain when specific muscle or the joint in relation to that muscle is used, weakness of the muscle or tendons, and an inability to use the muscle at all.

In some embodiments of this and other aspects described herein, the damaged muscle tissue results from muscle atrophy/wasting.

In some embodiments of this and other aspects described herein, the damaged muscle tissue results from sarcopenia. As used herein, the term "sarcopenia" refers to the loss of muscle mass and function that inevitably occurs with aging. Sarcopenia is responsible for decreased levels of physical activity which, in turn, can result in increased body fat and a further loss of muscle. Loss of muscle mass results from a negative net balance between muscle protein synthesis and muscle protein breakdown. The etiology of this loss of skeletal muscle mass and function is not believed to be clear. Reduced levels of physical activity, loss of motor units secondary to changes in the central nervous system, and inadequate protein intake have all been implicated

In some embodiments of this and other aspects described herein, the damaged muscle tissue results from a physical injury.

In some embodiments of this and other aspects of the invention, the damaged muscle is skeletal muscle.

In some embodiments, the subject has or is otherwise affected by muscle injury, insult or disease.

In some embodiments of this and other aspects described herein, disease resulting in damaged muscle tissue is a myopathy. Without limitation, myopathy can be a congenital myopathy or an acquired myopathy. Exemplary myopathies include, but are not limited to, dystrophies, myotonia (neuromytonia), congenital myopathies (e.g., nemaline myopathy, multi/minicore myopathy, centronuclear myopathy (or myotubular myopathy)), mitochondrial myopathies, familial periodic paralysis, inflammatory myopathies, metabolic myopathies (e.g., glycogen storage disease and lipid storage disorder), dermatomyositis, polymyositis inclusion body myositis, myositis ossificans, rhabdomyolysis and myoglobinuirias.

In some embodiments of this and other aspects described herein, myopathy is a dystrophy selected from the group consisting of muscular dystrophy, Duchenne muscular dystrophy, Becker's muscular dystrophy, Reflex sympathetic dystrophy, Retinal dystrophy, Conal dystrophy, Myotonic dystrophy, Corneal dystrophy, and any combinations thereof.

Congenital myopathy is a term sometimes applied to hundreds of distinct neuromuscular disorders that may be present at birth, but it is usually reserved for a group of rare inherited primary muscle disorders that cause hypotonia and weakness at birth or during the neonatal period and, in some cases, delayed motor development later in childhood. Patients suffer from weakness ranging from mild (late childhood onset and ability to walk through adulthood) to severe (respitatory insufficiency and death within the first year of life).

The most common types of congenital myopathy are nemaline myopathy, myotubular myopathy, central core myopathy, congenital fiber type disproportion, and multicore myopathy. They are distinguished primarily by their histological features, symptoms, and prognosis. Diagnosis is indicated by characteristic clinical findings and confirmed by muscle biopsy.

A therapeutically effective amount of a compound described herein can be for administration to a subject to treat any disease in which small muscles (e.g., sphincter muscles) are affected. A therapeutically effective amount of a compound described herein can be for administration to a subject to treat esophageal diseases (e.g., esophageal reflux disease and other diseases resulting from the loss of esophageal muscle control, tone or motility). A therapeutically effective amount of a compound described herein can be for administration to a subject to treat urinary incontinence. A therapeutically effective amount of a compounds described herein can be for administration to a subject to treat fecal incontinence. A therapeutically effective amount of a compound contemplated herein may be for administration to a subject to increase or improve spincter muscle tone.

*X-Linked Myotubular myopathy (XLMTM):* Myotubular myopathy is autosomal or X-linked. The more common autosomal variation produces mild weakness and hypotonia in both sexes. The X-linked variation affects males and results in severe skeletal muscle weakness and hypotonia, facial weakness, impaired swallowing, and respiratory muscle weakness and respiratory failure. However, female carriers rarely express significant clinical symptoms. Most patients die within the first year of life from respiratory failure. Some patients survive for several years and may show spontaneous improvement of respiratory function after birth. XLMTM has also been referred to as CNM, MTMX, X-linked centronuclear myopathy, and XMTM in the art.

The characteristic muscle histopathology consists of small rounded muscle fibers with centrally located nuclei surrounded by a halo devoid of contractile elements but containing mitochondria. The *MTM1* gene is mutated in vast majority of XLMTM patients. This gene is ubiquitously expressed and shows a muscle-specific alternative transcript due to the use of a different polyadenylation signal. Over one hundred thirty-three different disease associated mutations have been described in the *MTM1* gene. A list of *MTM1* mutations is maintained on the web at the Human Gene Mutation Database under entry for XLMTM and can be accessed at the following address: www.uwcm.ac.uk/uwcm/mg/search/119439.html. Mutations in the *MTM1* gene are widespread throughout the gene, although more have been found in exon 4, 12, 3, 8, 9, and 11, in that order when comparing the number of mutations to the nucleotide-length ratio for each exon. For a review of *MTM1* mutations in X-Linked Myotubular Myopathy see J. Laporte et al. 2000, 15:393-409.

Mutations in the *MTM1* gene include missense, nonsense, small insertions or deletions, large deletions and splice-site mutations. Most point mutations are truncating; however about 25% of the mutations are missense. While most truncating and splice mutations are associated with a severe phenotype, some missense mutations are associated with milder or less severe phenotype and prolonged survival.

*Nemaline myopathy:* Nemaline myopathy can be autosomal dominant or recessive and result from various mutations on different chromosomes. Nemaline myopathy may be severe, moderate, or mild in neonates. Severely affected patients may experience weakness of respiratory muscles and respiratory failure. Moderate disease produces progressive weakness in muscles of the face, neck, trunk, and feet, but life expectancy may be nearly normal. Mild disease is nonprogressive, and life expectancy is normal.

*Central core myopathy:* Inheritance is autosomal dominant. Most affected patients develop hypotonia and mild proximal muscle weakness as neonates. Many also have facial weakness. Weakness is nonprogressive, and life expectancy is normal. However, patients are at higher risk of developing malignant hyperthermia (the gene associated with central core myopathy is also associated with increased susceptibility to malignant hyperthermia).

*Congenital fiber type disproportion:* Congenital fiber type disproportion is inherited, but the pattern is poorly understood. Hypotonia and weakness of the face, neck, trunk, and limbs are often accompanied by skeletal abnormalities and dysmorphic features. Most affected children improve with age, but a small percentage develops respiratory failure.

*Multicore myopathy:* Multicore myopathy is usually autosomal recessive but may be autosomal dominant. Infants typically present with proximal weakness, but some children present later with generalized weakness. Progression is highly variable.

In some embodiments, a method described herein also comprises the step of diagnosing a subject for congenital myopathy before treating the subject with a compound described herein. A subject can be diagnosed for congenital myopathy based on the symptoms presented by the subject.

Generally, symptoms of congenital myopathies include, but are not limited to, depressed reflexes, enlarged muscles, difficulty relaxing muscles following contractions, stiff muscles, and rigid muscles. Specific symptoms are described below.

Central core disease is characterized by a mild, non-progressive muscle weakness. Signs of central core disease usually appear in infancy or early childhood and may present even earlier. There may be decreased fetal movements and breech (feet first) presentation *in utero*. The main features of CCD are poor muscle tone (hypotonia), muscle weakness, and skeletal problems including congenital hip dislocation, scoliosis (curvature of the spine), *pes cavus* (high-arched feet), and clubbed feet. Children with CCD experience delays in reaching motor milestones and tend to sit and walk much later than those without the disorder. A child with the disease usually cannot run easily, and may find that jumping and other physical activities are often impossible. Although central core disease may be disabling, it usually does not affect intelligence or life expectancy.

People who have central core disease are sometimes vulnerable to malignant hyperthermia (MH), a condition triggered by anesthesia during surgery. MH causes a rapid, and sometimes fatal, rise in body temperature, producing muscle stiffness.

There is variability in age of onset, presence of symptoms, and severity of symptoms in nemaline myopathy (NM). Most commonly, NM presents in infancy or early childhood with weakness and poor muscle tone. In some cases there may have been pregnancy complications such as polyhydramnios (excess amniotic fluid) and decreased fetal movements. Affected children with NM tend to have delays in motor milestones such as rolling over, sitting and walking. Muscle weakness commonly occurs in the face, neck and upper limbs. Over time, a characteristic myopathic face (a long face that lacks expression) develops. Skeletal problems including chest deformities, scoliosis, and foot deformities may develop. In the most severe cases of NM, feeding difficulties and potentially fatal respiratory problems may also occur. In those who survive the first two years of life, muscle weakness tends to progress slowly or not at all.

Typically the X-linked form of MTM (XLMTM) is the most severe of the three forms (X-linked, autosomal recessive, and autosomal dominant). XLMTM usually presents as a newborn male with poor muscle tone and respiratory distress. The pregnancy may have been complicated by polyhydramnios and decreased fetal movements. Of those who survive the newborn period, many will at least partially depend on a ventilator for breathing. Because of the risk of aspiration, many will also have a gastrostomy tube (G-tube). Boys with XLMTM can experience significant delays in achieving motor milestones and may not ever walk independently. They tend to be tall with a characteristic facial appearance (long, narrow face with a highly arched roof of the mouth and crowded teeth). Intelligence is generally not affected. Medical complications that may develop include: scoliosis, eye problems (eye muscle paralysis and droopy eyelids), and dental malocclusion (severe crowding). In XLMTM, other problems including undescended testicles, spherocytosis, peliosis, elevated liver enzymes, and gallstones may occur.

The autosomal recessive and autosomal dominant forms of MTM tend to have a milder course than the X-linked form. The autosomal recessive form can present in infancy, childhood, or early adulthood. Common features include generalized muscle weakness with or without facial weakness and ophthalmoplegia (paralysis of the eye muscles). Although feeding and breathing problems can occur, affected individuals usually survive infancy. Onset of the autosomal dominant form ranges from late childhood through early adulthood. It tends to be the mildest of the three forms of MTM. Unlike the X-linked form of the condition, problems with other organs (such as the liver, kidneys, and gall bladder) haven't been reported with the autosomal recessive and autosomal dominant forms of MTM.

Diagnosis of a congenital myopathy generally includes evaluation of the subject's personal and family history, physical and neurological examinations that test reflexes and strength, and specialized tests. Since there is overlap between the symptoms of a congenital myopathy and other neuromuscular disorders, a number of tests may be performed to help narrow down the diagnosis. Serum CK (creatinine kinase) analysis, EMG (electromyelogram), nerve conduction studies, and muscle ultrasound tend to be of limited value in making this diagnosis. The definitive diagnosis of a congenital myopathy usually relies upon genetic testing and/or muscle biopsy. Also, muscle biopsy can be used to determine a patient's susceptibility to malignant hyperthermia.

*X-linked myotubular myopathy:* Diagnosis of X-linked MTM is usually made on muscle biopsy. Findings include: centrally located nuclei in muscle fibers that look like myotubules, absence of structures known as myofibrils, and possibly, persistence of certain proteins usually seen in fetal muscle cells. Gene testing detects a mutation (disease-causing gene change) in up to 97-98% of people with the X-linked form. Gene testing can comprise: (i) complete gene sequencing of the *MTM1* gene; (ii) mutation screening (scanning) by methods such as single-stranded conformational polymorphism (SSCP) or denaturing gradient high-performance liquid chromatography (DHPLC), followed by sequencing of the abnormal fragments; and (iii) deletion testing. XLMTM can also be diagnosed by measuring levels of myoubilarin. Patients with known *MTM1* mutations usually show abnormal myotubularin levels.

*Central core disease:* The muscle biopsy from a person with CCD typically displays a metabolically inactive "core" or central region that appears blank when stained (tested) for certain metabolic enzymes (proteins) that should be there. These central regions also lack mitochondria, the energy producing "factories" of the cells. Genetic testing for *RYR1* mutations is available on a research basis. The same genetic test may be used to determine the presence of the gene change in family members who may have or be at-risk for the disease. For families in which a *RYR1* mutation has been found, prenatal diagnosis may be possible using the DNA of fetal cells obtained from chorionic villus sampling (CVS) or amniocentesis.

*Nemaline myopathy:* The clinical diagnosis of NM is suspected in an infant under age one with muscle weakness and hypotonia (decreased muscle tone). Definitive diagnosis of nemaline myopathy is made by demonstration of nemaline bodies, rod-shaped structures characteristic of this disease, using a specific stain known as "Gomori trichrome" on a muscle biopsy sample. Muscle biopsy may also show predominance of structures known as type I fibers. Genetic testing is available on a clinical basis for one gene, the ACTA1 gene located on the long arm of chromosome 1. About 15% of NM cases are due to mutations in this gene. Prenatal diagnosis is possible for families with known ACTA1 mutations. The DNA of a fetus can be tested using cells obtained from chorionic villus sampling (CVS) or amniocentesis.

### Pharmaceutical Compositions

For administration to a subject, the proliferation enhancers can be provided in pharmaceutically acceptable compositions. These pharmaceutically acceptable compositions comprise a therapeutically-effective amount of one or more of the proliferation enhancers, formulated together with one or more pharmaceutically acceptable carriers (additives) and/or diluents. As described in detail below, the pharmaceutical compositions of the present invention can be specially formulated for administration in solid or liquid form, including those adapted for the following: (1) oral administration, for example, drenches (aqueous or non-aqueous solutions or suspensions), gavages, lozenges, dragees, capsules, pills, tablets (e.g., those targeted for buccal, sublingual, and systemic absorption), boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; (8) transmucosally; or (9) nasally. Additionally, compounds can be implanted into a patient or injected using a drug delivery system. See, for example, Urquhart, et al., Ann. Rev. Pharmacol. Toxicol. 24: 199-236 (1984); Lewis, ed. "Controlled Release of Pesticides and Pharmaceuticals" (Plenum Press, New York, 1981); U.S. Pat. No. 3,773,919; and U.S. Pat. No. 35 3,270,960.

As used here, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used here, the term "pharmaceutically-acceptable carrier" means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol (PEG); (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; (22) C₂-C₁₂ alcohols, such as ethanol; and (23) other non-toxic compatible substances employed in pharmaceutical formulations. Wetting agents, coloring agents, release agents, coating agents, sweetening agents, flavoring agents, perfuming agents, preservative and antioxidants can also be present in the formulation. The terms such as "excipient", "carrier", "pharmaceutically acceptable carrier" or the like are used interchangeably herein.

The phrase "therapeutically-effective amount" as used herein means that amount of a compound, material, or composition comprising a compound described herein which is effective for producing some desired therapeutic effect in at least a sub-population of cells in an animal at a reasonable benefit/risk ratio applicable to any medical treatment. For example, an amount of a compound administered to a subject that is sufficient to produce a statistically significant, measurable satellite cell proliferation or muscle repair or regeneration.

As used herein, the term "repair" refers to a process by which the damages of a muscle tissue are alleviated or completely eliminated. In some embodiments, at least one symptom of muscle tissue damage is alleviated by at least 5%, at least 10%, at least 20%, at least 30%, at least 40%, or at least 50%.

Determination of a therapeutically effective amount is well within the capability of those skilled in the art. Generally, a therapeutically effective amount can vary with the subject's history, age, condition, sex, as well as the severity and type of the medical condition in the subject, and administration of other pharmaceutically active agents.

As used herein, the term "administer" refers to the placement of a composition into a subject by a method or route which results in at least partial localization of the composition at a desired site such that desired effect is produced. Routes of administration suitable for the methods of the invention include both local and systemic administration. Generally, local administration results in more of the administered proliferation enhancer (or proliferation enhancer treated satellite cells) being delivered to a specific location as compared to the entire body of the subject, whereas, systemic administration results in delivery of the proliferation enhancer (or proliferation enhancer treated satellite cells) to essentially the entire body of the subject. One method of local administration is by intramuscular injection.

In the context of administering a compound treated cell, the term "administering" also include transplantation of such a cell in a subject. As used herein, the term "transplantation" refers to the process of implanting or transferring at least one cell to a subject. The term "transplantation" includes, e.g., autotransplantation (removal and transfer of cell(s) from one location on a patient to the same or another location on the same patient), allotransplantation (transplantation between members of the same species), and xenotransplantation (transplantations between members of different species). Skilled artisan is well aware of methods for implanting or transplantation of cells for muscle repair and regeneration, which are amenable to the present invention. See for example, U.S. Pat. No. 7,592,174 and U.S. Pat. Pub. No. 2005/0249731.

Furthermore, the proliferation enhancers can be formulated in the form of ointments, creams powders, or other formulations suitable for topical formulations. Without wishing to be bound by a theory, these formulations can deliver the proliferation enhancer from skin to deeper muscle tissue. Accordingly, such formulations can comprise one or more agents that enhance penetration of active ingredient through skin. For topical applications, the proliferation enhancer can be included in wound dressings and/or skin coating compositions.

A proliferation enhancer or composition comprising same can be administered by any appropriate route known in the art including, but not limited to, oral or parenteral routes, including intravenous, intramuscular, subcutaneous, transdermal, airway (aerosol), pulmonary, nasal, rectal, and topical (including buccal and sublingual) administration.

Exemplary modes of administration include, but are not limited to, injection, infusion, instillation, inhalation, or ingestion. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In preferred embodiments of the aspects described herein, the compositions are administered by intravenous infusion or injection.

As used herein, a "subject" means a human or animal. Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. Patient or subject includes any subset of the foregoing, e.g., all of the above, but excluding one or more groups or species such as humans, primates or rodents. In certain embodiments of the aspects described herein, the subject is a mammal, e.g., a primate, e.g., a human. The terms, "patient" and "subject" are used interchangeably herein. The terms, "patient" and "subject" are used interchangeably herein. A subject can be male or female.

In certain aspects, the subject does not have or is not otherwise affected by acute myeloid leukemia (AML). In certain aspects, the subject does not have or is not otherwise affected by cancer.

Preferably, the subject is a mammal. The mammal can be a human, non-human primate, mouse, rat, dog, cat, horse, or cow, but are not limited to these examples. Mammals other than humans can be advantageously used as subjects that represent animal models of disorders associated with autoimmune disease or inflammation. In addition, the methods and compositions described herein can be used to treat domesticated animals and/or pets.

A subject can be one who has been previously diagnosed with or identified as suffering from or having a disorder characterized with muscle damage or muscle atrophy/wasting.

A subject can be one who is not currently being treated with a compound described herein.

A subject can be one who has been previously diagnosed with a disease that is being treated with a therapeutic regime comprising compound described herein, wherein the disease is not a disease characterized with muscle damage or muscle atrophy/wasting

The treatment method may comprise adjusting the therapeutic regime of the subject such that at least one symptom of muscle damage is reduced. Without limitation, a therapeutic regime can be adjusted by modulating the frequency of administration of the proliferation enhancer and/or by altering the site or mode of administration.

In some embodiments of the aspects described herein, the use of the compound for muscle repair or regeneration further comprises diagnosing a subject for muscle damage or muscle atrophy/wasting before treating the subject for muscle repair or regeneration.

In some embodiments of the aspects described herein, the use of the compound for muscle repair or regeneration further comprises selecting a subject with muscle damage or muscle atrophy/wasting before treating the subject for muscle repair or regeneration.

A compound described herein can be co-administrated to a subject in combination with a pharmaceutically active agent. Exemplary pharmaceutically active compound include, but are not limited to, those found in Harrison's Principles of Internal Medicine, 13th Edition, Eds. T.R. Harrison et al. McGraw-Hill N.Y., NY; Physicians' Desk Reference, 50th Edition, 1997, Oradell New Jersey, Medical Economics Co.; Pharmacological Basis of Therapeutics, 8th Edition, Goodman and Gilman, 1990; United States Pharmacopeia, The National Formulary, USP XII NF XVII, 1990; current edition of Goodman and Oilman's The Pharmacological Basis of Therapeutics; and current edition of The Merck Index.

The pharmaceutically active agent may be a growth factor. Exemplary growth factors include, but are not limited to, basic epidermal growth factor (bEGF), fibroblast growth factors (FGF), FGF-1, FGF-2 (bFGF), FGF-4, thymosins, platelet-derived growth factors (PDGF), insulin binding growth factors (IGF), IGF-1, IGF-2, epidermal growth factor (EGF), transforming growth factor (TGF), TGF-alpha, TGF-beta, cartilage inducing factors-A and -B, osteoid-inducing factors, osteogenin, bone morphogenic proteins, and other bone growth factors, collagen growth factors, heparin-binding growth factor-1 or -2, and their biologically active derivatives.

The proliferation enhancer and the pharmaceutically active agent can be administrated to the subject in the same pharmaceutical composition or in different pharmaceutical compositions (at the same time or at different times). When administrated at different times, the proliferation enhancer and the pharmaceutically active agent can be administered within 5 minutes, 10 minutes, 20 minutes, 60 minutes, 2 hours, 3 hours, 4, hours, 8 hours, 12 hours, 24 hours of administration of the other. When the proliferation enhancerand the pharmaceutically active agent are administered in different pharmaceutical compositions, routes of administration can be different.

The amount of the proliferation enhancer that can be combined with a carrier material to produce a single dosage form will generally be that amount of the proliferation enhancer that produces a therapeutic effect. Generally out of one hundred percent, this amount will range from about 0.01% to 99% of the compound, preferably from about 5% to about 70%, most preferably from 10% to about 30%.

Toxicity and therapeutic efficacy can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compositions that exhibit large therapeutic indices, are preferred.

As used herein, the term ED denotes effective dose and is used in connection with animal models. The term EC denotes effective concentration and is used in connection with in vitro models.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized.

The therapeutically effective dose can be estimated initially from cell culture assays. A dose may be formulated in animal models to achieve a circulating plasma concentration range that includes the IC50 (i.e., the concentration of the therapeutic which achieves a half-maximal inhibition of symptoms) as determined in cell culture. Levels in plasma may be measured, for example, by high performance liquid chromatography. The effects of any particular dosage can be monitored by a suitable bioassay.

The dosage may be determined by a physician and adjusted, as necessary, to suit observed effects of the treatment. Generally, the compositions are administered so that the proliferation enhancer is given at a dose from 1 µg/kg to 150 mg/kg, 1 µg/kg to 100 mg/kg, 1 µg/kg to 50 mg/kg, 1 µg/kg to 20 mg/kg, 1 µg/kg to 10 mg/kg, 1ug/kg to 1mg/kg, 100 µg/kg to 100 mg/kg, 100 µg/kg to 50 mg/kg, 100 µg/kg to 20 mg/kg, 100 µg/kg to 10 mg/kg, 100µg/kg to 1mg/kg, 1 mg/kg to 100 mg/kg, 1 mg/kg to 50 mg/kg, 1 mg/kg to 20 mg/kg, 1 mg/kg to 10 mg/kg, 10 mg/kg to 100 mg/kg, 10 mg/kg to 50 mg/kg, or 10 mg/kg to 20 mg/kg. It is to be understood that ranges given here include all intermediate ranges, for example, the range 1 tmg/kg to 10 mg/kg includes 1mg/kg to 2 mg/kg, 1mg/kg to 3 mg/kg, 1mg/kg to 4 mg/kg, 1mg/kg to 5 mg/kg, 1mg/kg to 6 mg/kg, 1mg/kg to 7 mg/kg, 1mg/kg to 8 mg/kg, 1mg/kg to 9 mg/kg, 2mg/kg to 10mg/kg, 3mg/kg to 10mg/kg, 4mg/kg to 10mg/kg, 5mg/kg to 10mg/kg, 6mg/kg to 10mg/kg, 7mg/kg to 10mg/kg, 8mg/kg to 10mg/kg, 9mg/kg to 10mg/kg , and the like. It is to be further understood that the ranges intermediate to the given above are also within the scope of this invention, for example, in the range 1mg/kg to 10 mg/kg, dose ranges such as 2mg/kg to 8 mg/kg, 3mg/kg to 7 mg/kg, 4mg/kg to 6mg/kg , and the like.

In some embodiments, the compositions are administered at a dosage so that the proliferation enhancer or a metabolite thereof has an *in vivo* concentration of less than 500 µM, less than 400 µM, less than 300 µM, less than 250 µM, less than 200 µM, less than 150 µM, less than 100 µM, less than 50 µM, less than 25 µM, less than 20 µM, less than 10 µM, less than 5µM, less than 1 µM, less than 0.5 µM, less than 0.1µM, than 500 nM, less than 400 nM, less than 300 nM, less than 250 nM, less than 200 nM, less than 150 nM, less than 100 nM, less than 50 nM, less than 25 nM, less than 20 nM, less than 10 nM less than 5nM, less than 1 nM, less than 0.5 nM, less than 0.1nM, less than 0.05 nM, less than 0.01 nM, less than 0.005 nM, less than 0.001 nM after 15 mins, 30 mins, 1 hr, 1.5 hrs, 2 hrs, 2.5 hrs, 3 hrs, 4 hrs, 5 hrs, 6 hrs, 7 hrs, 8 hrs, 9 hrs, 10 hrs, 11 hrs, 12 hrs or more of time of administration.

With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment or make other alteration to treatment regimen. The dosing schedule can vary from once a week to daily depending on a number of clinical factors, such as the subject's sensitivity to the polypeptides. The desired dose can be administered everyday or every third, fourth, fifth, or sixth day. The desired dose can be administered at one time or divided into subdoses, e.g., 2-4 subdoses and administered over a period of time, e.g., at appropriate intervals through the day or other appropriate schedule. Such sub-doses can be administered as unit dosage forms. In some embodiments of the aspects described herein, administration is chronic, e.g., one or more doses daily over a period of weeks or months. Examples of dosing schedules are administration daily, twice daily, three times daily or four or more times daily over a period of 1 week, 2 weeks, 3 weeks, 4 weeks, 1 month, 2 months, 3 months, 4 months, 5 months, or 6 months or more.

### Screening assays

Described herein is a method of screening for a candidate compound for stimulating or increasing proliferation in a satellite cell population, the method comprising:
(a) contacting a population of satellite cells with a test compound;
(b) assessing satellite proliferation; and
(c) selecting the compound that induces, increases or enhances satellite cell proliferation.

As used herein, the term "test compound" refers to compounds and/or compositions that are to be screened for their ability to induce, stimulate, enhance, or increase satellite cell proliferation. The test compounds can include a wide variety of different compounds, including chemical compounds and mixtures of chemical compounds, e.g., small organic or inorganic molecules; saccharines; oligosaccharides; polysaccharides; biological macromolecules, e.g., peptides, proteins, and peptide analogs and derivatives; antibodies, antibodies fragments, peptidomimetics; nucleic acids; nucleic acid analogs and derivatives; an extract made from biological materials such as bacteria, plants, fungi, or animal cells; animal tissues; naturally occurring or synthetic compositions; and any combinations thereof.

The number of possible test compounds runs into millions. Methods for developing small molecule, polymeric and genome based libraries are described, for example, in Ding, et al. J Am. Chem. Soc. 124: 1594-1596 (2002) and Lynn, et al., J. Am. Chem. Soc. 123: 8155-8156 (2001). Commercially available compound libraries can be obtained from, e.g., ArQule, Pharmacopia, graffinity, Panvera, Vitas-M Lab, Biomol International and Oxford. These libraries can be screened using the screening devices and methods described herein. Chemical compound libraries such as those from NIH Roadmap, Molecular Libraries Screening Centers Network (MLSCN) can also be used. A comprehensive list of compound libraries can be found at www.broad.harvard.edu/chembio/platform/screening/compound_libraries/index.htm. A chemical library or compound library is a collection of stored chemicals usually used ultimately in high-throughput screening or industrial manufacture. The chemical library can consist in simple terms of a series of stored chemicals. Each chemical has associated information stored in some kind of database with information such as the chemical structure, purity, quantity, and physiochemical characteristics of the compound.

Depending upon the particular embodiment being practiced, the test compounds can be provided free in solution, or may be attached to a carrier, or a solid support, e.g., beads. A number of suitable solid supports may be employed for immobilization of the test compounds. Examples of suitable solid supports include agarose, cellulose, dextran (commercially available as, i.e., Sephadex, Sepharose) carboxymethyl cellulose, polystyrene, polyethylene glycol (PEG), filter paper, nitrocellulose, ion exchange resins, plastic films, polyaminemethylvinylether maleic acid copolymer, glass beads, amino acid copolymer, ethylene-maleic acid copolymer, nylon, silk, etc. Additionally, for the methods described herein, test compounds may be screened individually, or in groups. Group screening is particularly useful where hit rates for effective test compounds are expected to be low such that one would not expect more than one positive result for a given group.

The test compound can induce, enhance, or increase satellite cell proliferation by at least 5%, 10%, 20%, 30%, 40%, 50%, 50%, 70%, 80%, 90%, 1-fold, 1.1-fold, 1.5-fold, 2-fold, 3-fold, 4-fold, 5-fold, 10-fold, 50-fold, 100-fold or more relative to an untreated control.

The step of assessing satellite cell proliferation may comprise detecting a satellite cell marker.

The step of assessing satellite cell proliferation may comprise detecting a satellite cell marker and a cell replication marker. A selected test compound can be further limited to the compound where the satellite cell marker and the cell-replication marker co-localize in the same cell.

Increased or enhanced satellite proliferation can be assessed by: (i) increased total number of cells in the culture, as compared to an untreated control; (ii) increased total number of cells expressing at least one satellite cell marker in the culture, as compared to an untreated control; (iii) increased ratio of cells expressing at least one satellite cell marker to the total number of cells in the culture, as compared to an untreated control; (iv) increased number of cells expressing at least one cell-replication marker, as compared to an untreated control; (v) increased ratio of cells expressing at least one cell-replication marker, as compared to an untreated control; or (vi) a combination thereof.

Satellite cell proliferation may be assessed via automated image acquisition and analysis using a Cellomics ArrayScan VTI. The acquisition thresholds/parameters are established such that the computer-based calls of replication events are consistent with human-based calls. Such automated image acquisition and analysis allows for high-throughput screening of compounds.

Generally plating density can range from about 10k cells/well to about 100k cells/well. Cellular plating density may be in the range from about 25k cells/well to about 75k cells/well, such as about 60k cells/well. Generally, at least 75%, 80%, 85%, 90%, 95% or more of the cells are viable at time of plating.

After plating, satellite cells can be allowed to adhere to the surface for a sufficient time, e.g. at least 1 hour, 2 hours, 3, hours, 4 hours, 6 hours, 8 hours, 12 hours, 24 hours, 36 hours, 48 hours or more, before contacting with the test compound. In some embodiments, the cells are allowed to adhere for 48 hours before compound treatment. After the cells have been allowed to adhere for a sufficient time, the media can be changed before treatment with compound of interest.

Generally, compounds can be tested at any concentration that can enhance replication of β-cells relative to a control over an appropriate time period. In some embodiments, compounds are tested at concentration in the range of about 0.1nM to about 1000mM. Preferably the compound is tested in the range of about 0.1µM to about 20µM, about 0.1µM to about 10µM, or about 0.1µM to about 5µM, such as 1 µM.

The satellite cell population can be maintained at any temperature suitable for satellite cell cultures. The satellite cells may be maintained at a temperature in the range of about 15°C to about 55°C, such as at 37°C.

Generally, the number of satellite cells in the culture can be counted after the satellite cells have been in contact with the test compound for a sufficient time, e.g., at least 1 hour, at least 2 hours, at least 3, hours, at least 4 hours, at least 5 hours, at least 6 hours, at least 7 hours, at least 8 hours, at least 9 hours, at least 10 hours, at least 11 hours, at least 12 hours, at least 24 hours, at least 36 hours, at least 48 hours, at least 5 days, at least 1 week, at least 2 weeks, at least 3 weeks, or more. The cells can be counted manually or by an automated system. Use of an automated system allows for high-throughput screening of compounds.

The inventors have discovered that in some instances prolonged treatment with the proliferation enhancer does not lead to higher satellite cell proliferation as compared to treatment for a shorter period of time. Accordingly, the number of satellite cells in the culture can be counted after the satellite cells have been in contact with the test compound for between 1 hour and seven days. For example, the number of satellite cells in the culture can be counted after the satellite cells have been in contact with the test compound for one, two, three, four, five or six days.

Satellite-cell and replication cell marker detection can be done after the satellite cells are in contact with the test compound for a sufficient time, e.g., at least 1 hour, at least 2 hours, at least 3, hours, at least 4 hours, at least 5 hours, at least 6 hours, at least 7 hours, at least 8 hours, at least 9 hours, at least 10 hours, at least 11 hours, at least 12 hours, at least 24 hours, at least 36 hours, at least 48 hours, at least 5 days, at least 1 week, at least 2 weeks, at least 3 weeks, or more. After marker detection, number of cells expressing cell-replication and/or satellite cell marker can be counted. Marker detection can include the steps of preparing the cells for the appropriate assay, e.g., fixing and/or staining the cells.

The satellite cell and cell replication marker detection can be done after the satellite cells are in contact with the test compound for 1 hour to about 7 days.

The satellite cell and cell replication marker detection can be done after the satellite cells are in contact with the test compound for one, two, three, four, five or six days.

The method may comprise additionally selecting the compound that increased the ratio of satellite cells to the total number of cells as compared to an untreated control.

The term "satellite-cell marker" refers to, without limitation, proteins, peptides, nucleic acids, polymorphism of proteins and nucleic acids, splice variants, fragments of proteins or nucleic acids, elements, and other analytes which are specifically expressed or present in satellite cells. Exemplary satellite cell markers include, but are not limited to, PAX7, PAX3, Myf5, MyoD, and desmin. Other markesr that can be used include, but are not limited to, beta-integrin 1 and CXCR4. Method of identifying satellite cells is also described in Sherwood et al. (Cell, 2004, 119: 543-554).

The terms "cell replication marker" refers to, without limitation, proteins, peptides, nucleic acids, polymorphisma of proteins and nucleic acids, splice variants, fragments of proteins or nucleic acids, elements, and other analytes which are specifically associated with cell proliferation. Additionally, "cell-replication marker", includes enzymatic activity when changes, e.g., increase or decrease, in the enzymatic activity are specifically associated with cell proliferation. Exemplary cell replication markers include, but are not limited to, phosphorylated histone H3 (PH3), Ki-67 protein, phosphorylated MPM-2 antigen, Proliferating Cell Nuclear Antigen (PCNA, a protein that is expressed in the nuclei of cells during the DNA synthesis phase of the cell cycle), phospho-S780-Rb epitope (Jacobberger, JW , et al. Cytometry A (2007), 73A:5-15), Cenp-F (mitosin), class III β-Tublin, spindal checkpint protine hMad2, phosphorylated myosin light chain kinase, topoisomerase II, Check point kinase 1 (Chk1), Vesicular Monoamine Transporter 2 (VMAT2), loss of cyclin-dependent kinase 1 (Cdk1) kinase activity. Histone H3 can be phosphorylated at Ser28 or Ser10.

Cell replication markers and satellite cell markers can be detected by methods known in the art and easily available to the skilled artisan, for example appropriate ELISA, immunofluourescent, or immunohistochemcial assays can be used for detection. MIB-1 is a commonly used monoclonal antibody that detects the Ki-67 protein. It is used in clinical applications to determine the *Ki-67 labelling index*. Ki-67 ELISA are described in Klein, CL, et al., J. Mater. Sci. Mater. Med. (2000), 11:125-132; Frahm, SO, et al., J. Immunol. Methods (199*0, 211:43-50; and Key G, et al., ,J. Immunol. Methods (1994), 177:113-117. Phospho-Histone H3 antibodies for detection of phosphorylated Histone H3 are commercially available from Cell Signaling Technology and Millipore. Antibodies against PCNA are commercially available from Sigma Aldrich. Antibodies to MPM-2 antigen are specific for cells in mitosis, recognizes a family of proteins that share a common phosphorylated epitope.

The satellite cell can be isolated from a mammal such as a mouse. Methods for isolating cells from a subject are described, for example in Sherwood et al. (Cell, 2004, 119: 543-554).

A satellite cell can be transformed cell. As used herein, the term "transformed cells" is art recognized and refers to cells which have converted to a state of unrestrained growth, i.e., they have acquired the ability to grow through an indefinite number of divisions in culture. Transformed cells may be characterized by such terms as neoplastic, anaplastic and/or hyperplastic, with respect to their loss of growth control. In general, term "transformed satellite cell" refers to satellite cells which exhibit increased capacity to persist in serial subcultures or increased growth rate *in vitro.*

The screening method may be a high-throughput screening. High- throughput screening (HTS) is a method for scientific experimentation that uses robotics, data processing and control software, liquid handling devices, and sensitive detectors. High- Throughput Screening or HTS allows a researcher to quickly conduct millions of biochemical, genetic or pharmacological tests. High-Throughput Screening are well known to one skilled in the art, for example, those described in U. S. Pat. Nos. 5,976,813; 6,472,144; 6,692,856; 6,824,982; and 7,091,048.

HTS uses automation to run a screen of an assay against a library of candidate compounds. An assay is a test for specific activity: usually inhibition or stimulation of a biochemical or biological mechanism. Typical HTS screening libraries or "decks" can contain from 100,000 to more than 2,000,000 compounds.

The key labware or testing vessel of HTS is the microtiter plate: a small container, usually disposable and made of plastic, that features a grid of small, open divots called wells. Modern microplates for HTS generally have either 384, 1536, or 3456 wells. These are all multiples of 96, reflecting the original 96 well microplate with 8 x 12 9mm spaced wells.

To prepare for an assay, the researcher fills each well of the plate with the appropriate reagents that he or she wishes to conduct the experiment with, such as a satellite cell population. After some incubation time has passed to allow the reagent to absorb, bind to, or otherwise react (or fail to react) with the compounds in the wells, measurements are taken across all the plate's wells, either manually or by a machine. Manual measurements are often necessary when the researcher is using microscopy to (for example) seek changes that a computer could not easily determine by itself. Otherwise, a specialized automated analysis machine can run a number of experiments on the wells such as colorimetric measurements, radioactivity counting, etc. In this case, the machine outputs the result of each experiment as a grid of numeric values, with each number mapping to the value obtained from a single well. A high-capacity analysis machine can measure dozens of plates in the space of a few minutes like this, generating thousands of experimental data points very quickly.

By way of example, the assay was performed as follows. Satellite cells were harvested from CAG-B-actin-GFP mice, aged 2-4 months old via the FACS isolation method outlined in Sherwood et al 2004 and Cerletti et al 2008. Briefly, all limb muscles and abdominal muscles were harvested from the animals and digested first in a 0.2% collagenase solution, then in a 0.0125% collagenase/ 0.05% dispase solution. The filtered solution was stained for cell surface markers, and put through FACS. Cells that were negative for Mac1, Sca1 and CD45 and positive for Beta-integrin 1 and CXCR4 were used for the screening assay. Cells were plated directly from the FACS machine at 50 cells/well, into 96-well plates coated with 10ug/mL laminin (4-6 hours at 37C, then partially removed). Media for the screen was Ham's F-10 supplemented with 10% heat-inactivated horse serum, 1X penicillin/streptomycin and 1X L-glutamine. Basic FGF (bFGF) was added only to the positive control wells; all other wells received media only. The day of plating was called Day 0. The day after plating, Day 1, compounds were added. All compounds were dissolved in DMSO, and initially tested at10uM, 1uM or 0.1uM in duplicate. The negative control for each plate was the same concentration of DMSO (no compounds dissolved). Compounds were incubated with the cells until Day 4, with no media change. bFGF was spiked into positive control wells daily. On Day 4, plates were fixed in 4% paraformaldehyde and washed with phosphate buffered saline. Plates were imaged directly on the Opera Confocal imager, since the cells were readily visible from the CAG-EGFP-Beta-actin transgene. An Acapella script was used to count the number of cells in each well. Wells were scored for proliferation based on the cell counts (DMSO-treated wells usually had around 50 cells at the end of the assay, bFGF-treated wells usually had 150-250 cells at the end). Inventors selected compounds as hits if they had cell numbers greater than or equal to one (or, for the second session of screening, three) standard deviation(s) from the DMSO controls. Compounds thus selected were then tested in an initial dose curve, usually concentrations between 20nM-30uM commensurate with the concentration flagged as a hit. A compound was active if it was able to cause proliferation to cell numbers at least two standard deviations over the DMSO negative control. Any compounds that were found to be active in the dose curve were re-supplied from the original vendors. Re-supplied compounds were again tested in a dose curve for proliferation ability. Compounds still found to be active were then put in the queue for optimization and characterization.

### Kits

A kit for muscle repair or regeneration may comprise a compound described herein, e.g., a compound selected from the group consisting of kinase inhibitors, G protein coupled receptor (GPCR) modulators, histone deacetylases (HDAC) modulators, hedgehog signaling pathway modulators, neuropeptides, dopamine receptor modulators, serotonin receptor modulators, histamine receptor modulators, ionophores, ion channel modulators, gamma-secretase modulators, and any combinations thereof. The compound can be pre-formulated into a pharmaceutical formulation for administration or ingredients for formulating into a pharmaceutical formulation can be provided in the kit.

The kit may comprise a compound described herein, wherein the compound is formulated for topical application.

The kit may comprise a population of satellite cells, wherein at least one cell in the population has been pretreated by contacting the cells with a compound described herein.

In addition to the above mentioned components, the kit can include informational material. The informational material can be descriptive, instructional, marketing or other material that relates to the methods described herein and/or the use of the compound for the methods described herein. For example, the informational material describes methods for administering the formulation to a subject. The kit can also include a delivery device.

The informational material can include instructions to administer the formulation in a suitable manner, e.g., in a suitable dose, dosage form, or mode of administration (e.g., a dose, dosage form, or mode of administration described herein). The informational material can include instructions for identifying a suitable subject, e.g., a human, e.g., an adult human. The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as Braille, computer readable material, video recording, or audio recording. The informational material of the kit may be a link or contact information, e.g., a physical address, email address, hyperlink, website, or telephone number, where a user of the kit can obtain substantive information about the formulation and/or its use in the methods described herein. Of course, the informational material can also be provided in any combination of formats.

The individual components of the formulation can be provided in one container. Alternatively, it can be desirable to provide the components of the formulation separately in two or more containers, *e.g*., one container for an oligonucleotide preparation, and at least another for a carrier compound. The different components can be combined, e.g., according to instructions provided with the kit. The components can be combined according to a method described herein, *e.g*., to prepare and administer a pharmaceutical composition.

In addition to the formulation, the composition of the kit can include other ingredients, such as a solvent or buffer, a stabilizer or a preservative, and/or a second agent for treating a condition or disorder described herein. Alternatively, the other ingredients can be included in the kit, but in different compositions or containers than the formulation. In such embodiments, the kit can include instructions for admixing the formulation and the other ingredients, or for using the oligonucleotide together with the other ingredients.

The compound can be provided in any form, e.g., liquid, dried or lyophilized form. It is preferred that the formulation be substantially pure and/or sterile. When the formulation is provided in a liquid solution, the liquid solution preferably is an aqueous solution, with a sterile aqueous solution being preferred. When the formulation is provided as a dried form, reconstitution generally is by the addition of a suitable solvent. The solvent, e.g., sterile water or buffer, can optionally be provided in the kit.

The kit may contain separate containers, dividers or compartments for the formulation and informational material. For example, the formulation can be contained in a bottle, vial, or syringe, and the informational material can be contained in a plastic sleeve or packet. The separate elements of the kit may be contained within a single, undivided container. For example, the formulation is contained in a bottle, vial or syringe that has attached thereto the informational material in the form of a label.

The kit may include a plurality, e.g., a pack, of individual containers, each containing one or more unit dosage forms of the formulation. For example, the kit includes a plurality of syringes, ampules, foil packets, or blister packs, each containing a single unit dose of the formulation. The containers of the kits can be air tight and/or waterproof.

### Some selected definitions

For convenience, certain terms employed herein, in the specification, examples and appended claims are collected herein. Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Further, unless otherwise required by context, singular terms shall include pluralities and plural terms shall include the singular.

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein.

As used herein the term "comprising" or "comprises" is used in reference to compositions, methods, and respective component(s) thereof, that are essential to the invention, yet open to the inclusion of unspecified elements, whether essential or not.

The singular terms "a," "an," and "the" include plural referents unless context clearly indicates otherwise. Similarly, the word "or" is intended to include "and" unless the context clearly indicates otherwise.

Other than in the operating examples, or where otherwise indicated, all numbers expressing quantities of ingredients or reaction conditions used herein should be understood as modified in all instances by the term "about." The term "about" when used in connection with percentages may mean ±5% of the value being referred to. For example, about 100 means from 95 to 105.

Although methods and materials similar or equivalent to those described herein can be used in the practice or testing of this disclosure, suitable methods and materials are described below. The term "comprises" means "includes." The abbreviation, "e.g." is derived from the Latin exempli gratia, and is used herein to indicate a non-limiting example. Thus, the abbreviation "e.g." is synonymous with the term "for example."

The terms "decrease" , "reduced", "reduction" , "decrease" or "inhibit" are all used herein generally to mean a decrease by a statistically significant amount. However, for avoidance of doubt, "reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (e.g. absent level as compared to a reference sample), or any decrease between 10-100% as compared to a reference level.

The terms "increased," "increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The term "statistically significant" or "significantly" refers to statistical significance and generally means at least two standard deviations (2SD) away from a reference level. The term refers to statistical evidence that there is a difference. It is defined as the probability of making a decision to reject the null hypothesis when the null hypothesis is actually true.

The term "modulator" refers to a compound that alters or elicits an activity of a molecule. For example, a modulator may cause an increase or decrease in the magnitude of a certain activity of a molecule compared to the magnitude of the activity in the absence of the modulator. A modulator may be an inhibitor, which decreases the magnitude of one or more activities of a molecule. An inhibitor may completely prevent one or more activities of a molecule. A modulator may be an activator, which increases the magnitude of at least one activity of a molecule. The presence of a modulator may result in an activity that does not occur in the absence of the modulator. Without limitations, a modulator can be selected from the group consisting of small or large organic or inorganic molecules; monosaccharides; disaccharides; trisaccharides; oligosaccharides; polysaccharides; biological macromolecules, e.g., proteins, peptides, peptide analogs and derivatives thereof, peptidomimetics, nucleic acids, nucleic acid analogs and derivatives, enzymes, antibodies, portion or fragments of antibodies; an extract made from biological materials such as bacteria, plants, fungi, or animal cells or tissues; naturally occurring or synthetic compositions; and any combinations thereof.

The term "selective modulator" refers to a compound that selectively modulates a target activity.

The term "selectively modulates" refers to the ability of a selective modulator to modulate a target activity to a greater extent than it modulates a non-target activity.

The term "target activity" refers to a biological activity capable of being modulated by a modulator. Certain exemplary target activities include, but are not limited to, binding affinity, signal transduction, enzymatic activity, and the like.

The term "agonist" refers to a compound, the presence of which results in a biological activity of a receptor that is the same as the biological activity resulting from the presence of a naturally occurring ligand for the receptor.

The term "partial agonist" refers to a compound the presence of which results in a biological activity of a receptor that is of the same type as that resulting from the presence of a naturally occurring ligand for the receptor, but of a lower magnitude.

The term "antagonist" refers to a compound, the presence of which results in a decrease in the magnitude of a biological activity of a receptor. The presence of an antagonist may result in complete inhibition of a biological activity of a receptor.

The term "inhibitor" refers to molecules or substances or compounds or compositions or agents or any combination which are capable of inhibiting and/or reducing the activity of the target molecule. As used herein, the term "inhibitor" is interchangeable with the term "antagonist". The term "inhibitor" comprises competitive, non-competitive, functional and chemical antagonists. The term "partial inhibitor" means a molecule or substance or compound or composition or agent or any combination thereof that is capable of incompletely blocking the action of agonists through, inter alia, a non-competitive mechanism.

As used herein, the term "ligand" refers to an agent that binds to a target molecule. A ligand is not limited to an agent that binds to a recognized functional region of the target molecule, e.g., the active site of an enzyme, the antigen-combining site of an antibody, the hormone-binding site of a receptor, a cofactor-binding site, and the like. A ligand can also be an agent that binds any surface or conformational domains of the target compound. Therefore, the ligands encompass agents that in and of themselves may have no apparent or known biological function, beyond their ability to bind to the target in the manner described above. The term ligand encompasses agents that react upon binding and agents that do not react other than by binding.

As used herein, the term "small molecule" can refer to compounds that are "natural product-like," however, the term "small molecule" is not limited to "natural product-like" compounds. Rather, a small molecule is typically characterized in that it contains several carbon-carbon bonds, and has a molecular weight of less than 5000 Daltons (5 kD), preferably less than 3 kD, still more preferably less than 2 kD, and most preferably less than 1 kD. In some cases it is preferred that a small molecule have a molecular weight equal to or less than 700 Daltons.

The disclosure is further illustrated by the following examples which should not be construed as limiting. The examples are illustrative only, and are not intended to limit, in any manner, any of the aspects described herein. The following examples do not in any way limit the invention.

### EXAMPLES

### Example 1 - Screening assay

Satellite cells were harvested from CAG-B-actin-GFP mice, aged 2-4 months old via the FACS isolation method outlined in Sherwood et al 2004 and Cerletti et al 2008. Briefly, all limb muscles and abdominal muscles were harvested from the animals and digested first in a 0.2% collagenase solution, then in a 0.0125% collagenase/ 0.05% dispase solution. The filtered solution was stained for cell surface markers, and put through FACS. Cells that were negative for Mac1, Sca1 and CD45 and positive for Beta-integrin 1 and CXCR4 were used for the screening assay. Cells were plated directly from the FACS machine at 50 cells/well, into 96-well plates coated with 10ug/mL laminin (4-6 hours at 37C, then partially removed). Media for the screen was Ham's F-10 supplemented with 10% heat-inactivated horse serum, 1X penicillin/streptomycin and 1X L-glutamine. Basic FGF (bFGF) was added only to the positive control wells; all other wells received media only. The day of plating was called Day 0. The day after plating, Day 1, compounds were added. All compounds were dissolved in DMSO, and initially tested at 10uM, 1uM or 0.1uM in duplicate. The negative control for each plate was the same concentration of DMSO (no compounds dissolved). Compounds were incubated with the cells until Day 4, with no media change. bFGF was spiked into positive control wells daily. On Day 4, plates were fixed in 4% paraformaldehyde and washed with phosphate buffered saline. Plates were imaged directly on the Opera Confocal imager, since the cells were readily visible from the CAG-EGFP-Beta-actin transgene. An Acapella script was used to count the number of cells in each well. Wells were scored for proliferation based on the cell counts (DMSO-treated wells usually had around 50 cells at the end of the assay, bFGF-treated wells usually had 150-250 cells at the end). We selected compounds as hits if they had cell numbers greater than or equal to one (or, for the second session of screening, three) standard deviation(s) from the DMSO controls. Compounds thus selected were then tested in an initial dose curve, usually concentrations between 20nM-30uM commensurate with the concentration flagged as a hit. We called compounds active if they were able to cause proliferation to cell numbers at least two standard deviations over the DMSO negative control. Any compounds that were found to be active in the dose curve were re-supplied from the original vendors. Re-supplied compounds were again tested in a dose curve for proliferation ability. Compounds still found to be active were then put in the queue for optimization and characterization.

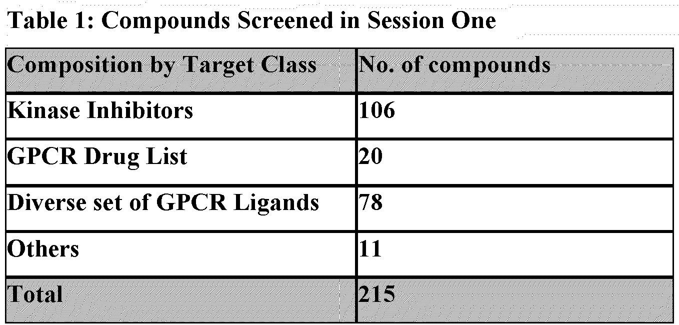

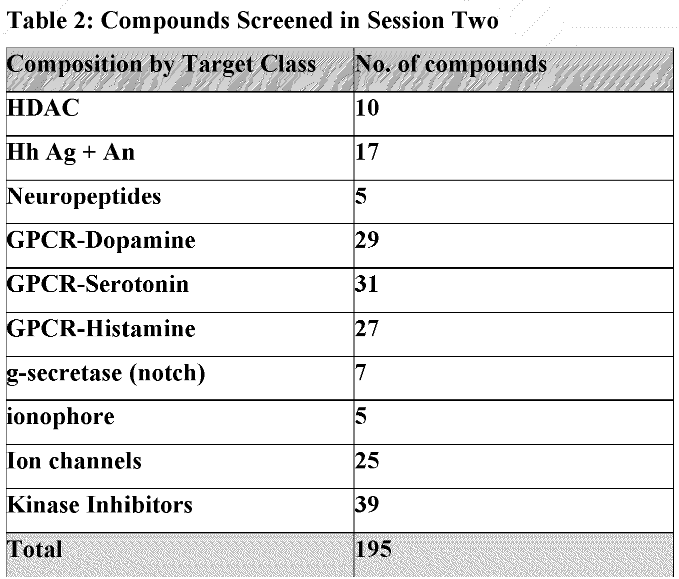

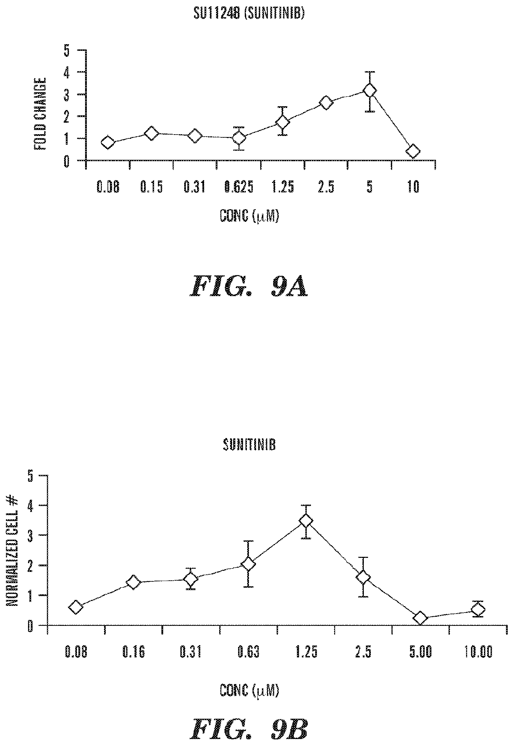

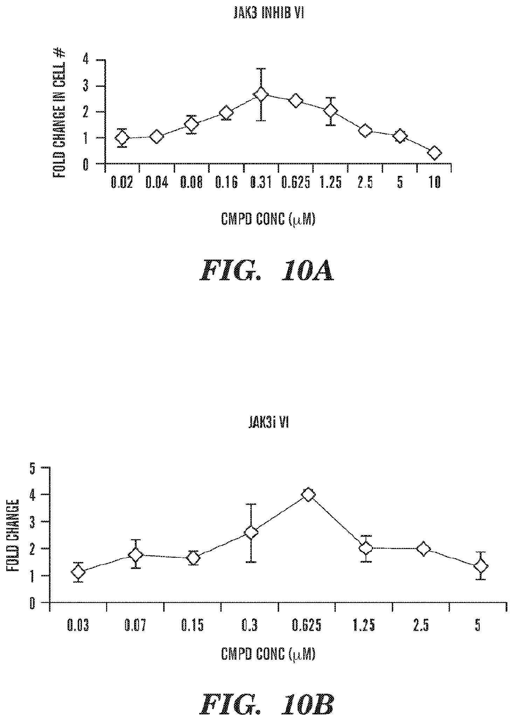

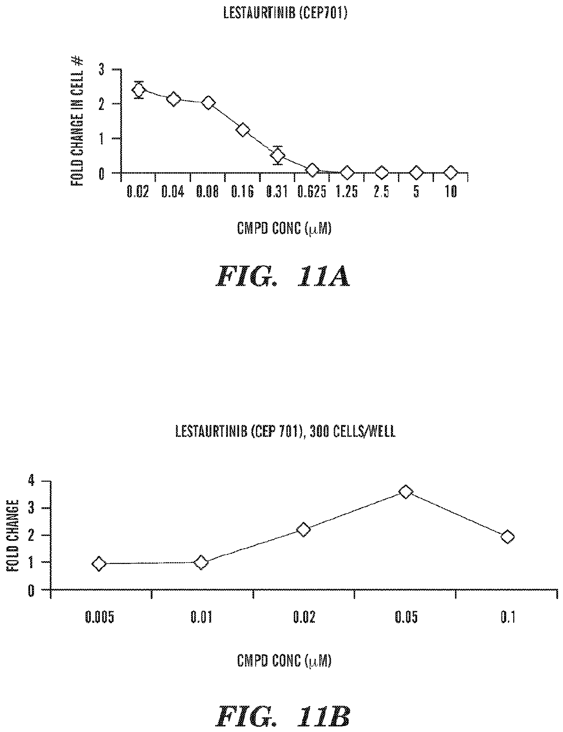

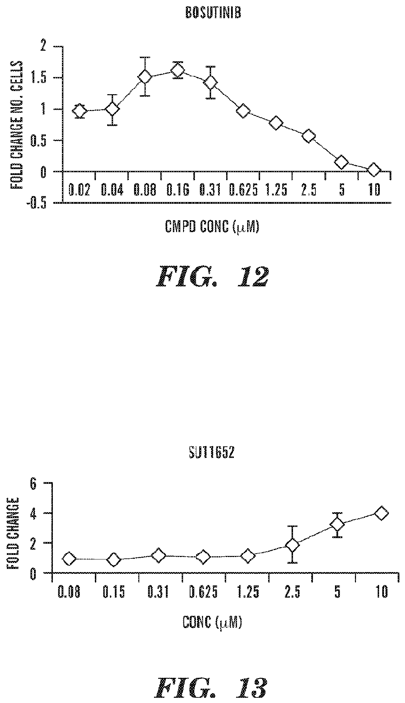

As described above, the inventors screened a set of 215 compounds comprised primarily of kinase inhibitors and GPCR ligands. The inventors assayed these compounds at 1µM, and counted as potential hits those compounds that scored greater than one standard deviation over the DMSO-treated negative control. Using these criteria, the inventors identified 20 compounds from the set as hits. The inventors then tested each of these 20 compounds in a dose response assay to validate their activity. Five compounds found to have activity using this method are shown for reference purposes in **Table 3.** Dose response curves for the five compounds are shown for reference purposes in **FIGS. 9A-13****.**

**Table 3:**

| **Compound** | **Library Source** | **Initial hit Conc.** | **Target** |
|---|---|---|---|
| Lestaurtinib (CEP-701) | Kinases | 1uM | Flt3 Kinase, Jak2, ROR gamma? |
| SU 11652 | Kinases | 1uM | PDGFR/EGFR |
| SU 11248 (Sunitinib) | Kinases | 1uM | EGFR, Flt3 kinase |
| Bosutinib (SKI 606) | Kinases | 1uM | Bcr-abl, SRC kinase |
| JAK3 Inhibitor VI | Kinases | 1uM | |

Three of the compounds (Sunitinib (SU11248), JAK3 inhibitor VI, and Lestaurtinib/CEP701) were retested and found to be active in the retest. Each compound promoted proliferation of the input SMP cells similar to that seen in the bFGF positive control (4.7±1.4 for Sunitinib and Lestaurtinib, 3.7±1.1 for JAK3 inhibitor VI). The DMSO-treated cells proliferated to a level of 1±0.3 in both trials.

In addition, the inventors also examined whether CEP 701 can synergize with bFGF to achieve even higher levels of SMP proliferation. For these experiments, 300 cells were plated in each well instead of the 50 cells/well used for the screen and subsequent dose response assays. This change in protocol was introduced in an effort to reduce the variability in the assays. Under these conditions 0.05µM CEP701 was added the day after plating the purified SMPs, and 5ng/mL bFGF was added to the appropriate samples on a daily basis. Three days after plating the media was changed and fresh compound was added. This additional change in the protocol was introduced both to improve viability and reduce variability. The plates were fixed four days after plating.

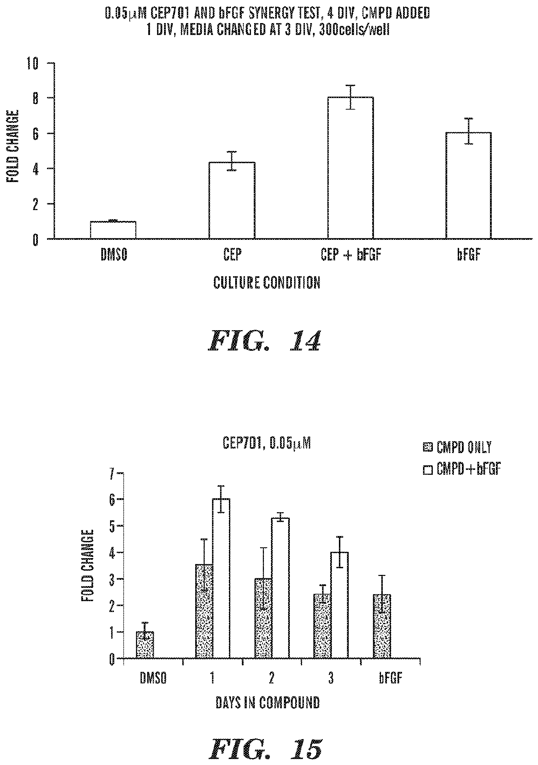

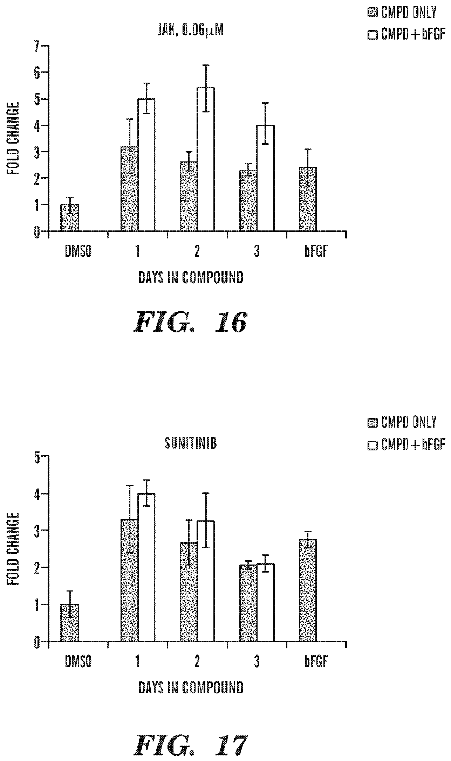

Cultures treated with both CEP701 and bFGF showed an 8-fold increase in proliferation (**FIG. 14**), indicating that CEP701 and bFGF can act additively to increase SMP expansion *in vitro.* Because changing the media seemed to lead to increased viability in the cells, the inventors decided to try removing the compounds several days before fixation to allow the cells to recover. For the following experiments, compounds were added the day after plating (Day 1). Compound was refreshed daily, until it was removed from the cultures completely on the day as indicated below. Cells were then cultured in media without supplemented compounds until fixation on Day 4. The inventors discovered that two days of recovery time was advantageous to the cultures. The inventors confirmed that discovered that CEP-701 can work additively with bFGF (**FIG. 15**). Additionally, while Jak3 inhibitor VI also worked additively with bFGF (**FIG. 16**), Sunitinib did not worked additively with bFGF (**FIG. 17**).

In order to assess the effects these compounds have on the identity of the SMPs, the inventors studied the ability of the treated SMPs to differentiate in culture. For these experiments, 300 cells were plated in each well at Day 0 in our standard proliferation media (10% horse serum in F-10). Compounds were added at Day 1 and SMPs were allowed to proliferate for three days. At Day 4, the media was switched to differentiation media (10% horse serum, 10%FBS, 0.5% chick embryo extract in high-glucose DMEM). Cultures were then incubated for three or four days under differentiation conditions and fixed on Day 7 or Day 8. They were then stained with Hoechst and anti-myosin heavy chain antibody.

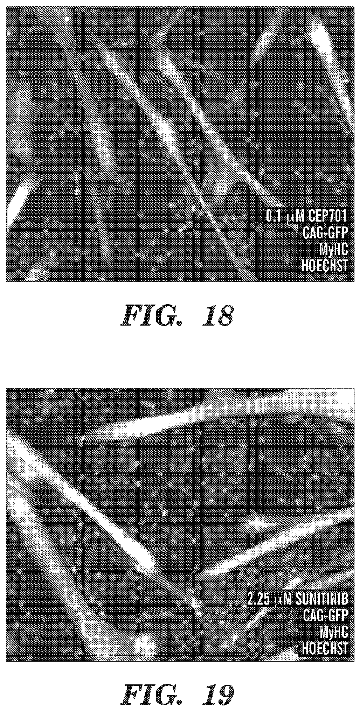

As shown in **FIGS. 18-20****,** treatment with CEP701, Sunitinib, or Jak3 inhibitor VI did not affect the ability of expanded SMPs to differentiate and fuse to form myotubes. Additionally, satellite cells that had been exposed to CEP701 for 3 days and then allowed to recover for two days before being put into differentiation conditions were also able to fuse into myotubes (data not shown).

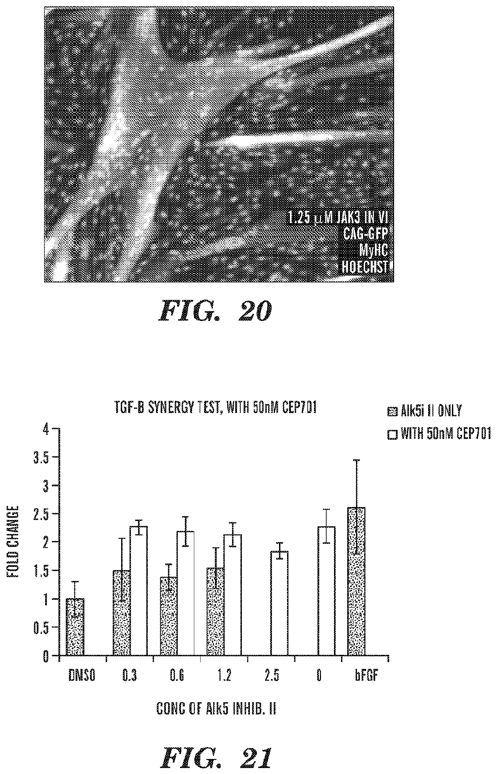

The inventors focused on CEP701 for further studies. The inventors next studied additive effect of CEP701 with TGF-beta inhibitors. TGF-Beta inhibitors are known to promote proliferation and prevent differentiation of satellite cells. Addition of Alk5 inhibitor II to the cultures produced a slight increase in proliferation. However, little additive effect was seen on addition of CEP701 and TGF-beta inhibitor **(****FIG. 21**).

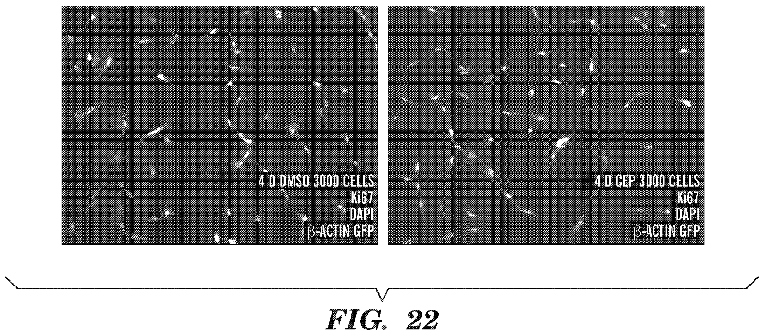

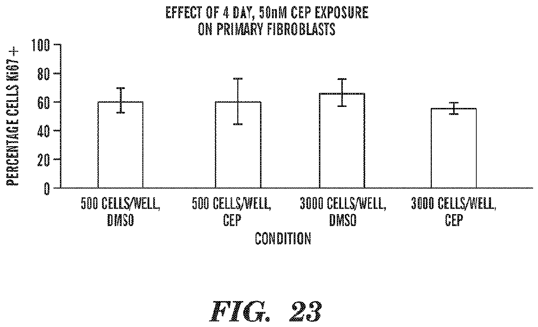

Next the inventors tested the specificity of CEP701 for proliferating satellite cells. The inventor isolated the Sca1-positive population of fibroblasts from the muscle preparation, obtained via FACS. Either 500 or 3000 fibroblasts were plated in each well, and cultured in DMEM supplemented with 10% FBS. Compound was added the day after plating, and refreshed daily. Plates were fixed five days after plating and stained for the proliferation marker Ki67. As seen in **FIG. 22****,** no difference in the percentage of proliferating cells between those exposed to CEP701 (right panel) and those exposed to DMSO (left panel) was seen. In addition, CEP701 had no effect on primary fibroblasts **(****FIG. 23**).

In another experiment, the inventors tested the CEP701 compound in aged tissue. The aged mice were 15 months old at the time of the experiment. The conditions used for the assay were the same as those used for young animals. After FACS, 300 cells/well were plated on Day 0 in the inventors' standard proliferation media (10% horse serum in F-10). Compound was added at Day 1 at indicated concentrations, and refreshed daily. At Day 4, compound was withdrawn and replaced with media only. Plates were fixed and imaged at Day 6.

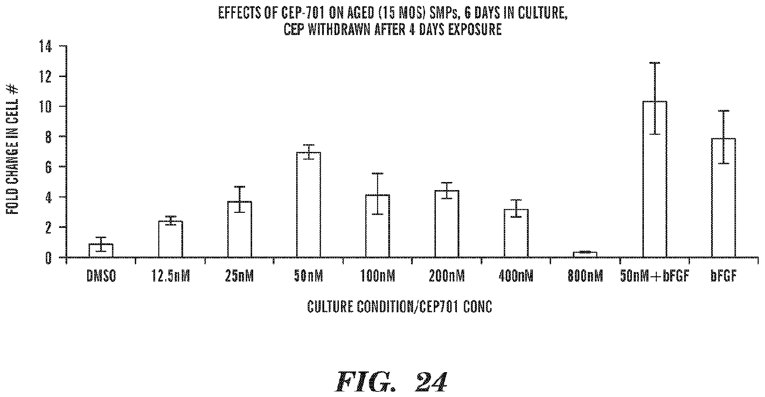

As seen in **FIGS. 24** and **25****,** 50nM CEP701 appeared to be the optimum concentration for aged cells as well as young cells. In both cases, exposure to 50nM CEP701 under the described conditions resulted in approximately a six-fold increase in the number of cells. CEP701 and bFGF were able to act additively in cultures of old cells, producing approximately a ten-fold increase in cell number.

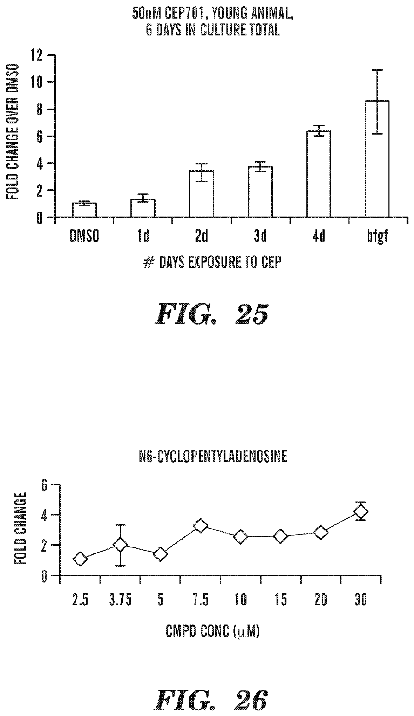

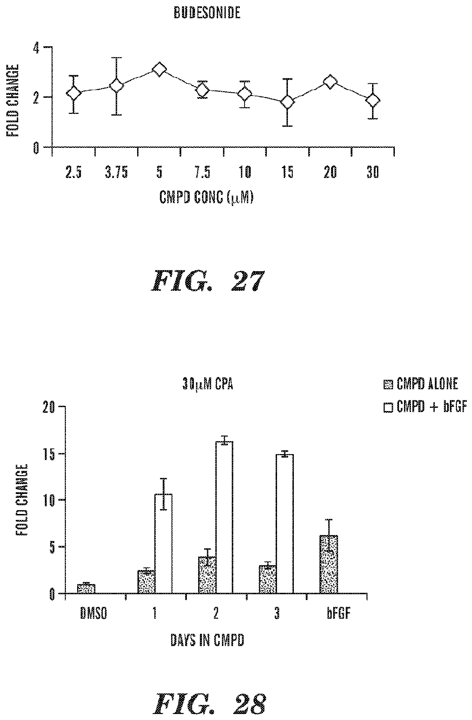

In another experiment, the inventors screened a new set of 200 compounds. This set was primarily focused on GPCR ligands, with some kinase inhibitors and other annotated compounds. The inventors screened the compounds at 10uM in duplicate, except the kinase inhibitors which were screened at 1uM in duplicate. Compounds were scored as potential hits if they increased proliferation of input SMPs at levels greater than three standard deviations over the negative control in either of the replicates. Using these criteria, the inventors identified and validated 2 hits (N6-cyclopentyladenosine, an adenosinse receptor agonist, and Budesonide, a glucocorticoid steroid) in subsequent dose response curves **(****FIGS. 26** and **27**).

After additional testing, we ultimately decided to resupply Budesonide, and N6-cyclopentyladenosine (CPA) from the original vendor in order to test a different batch of the compounds. We performed dose responses on all the re-supplied compounds, plating 50 cells in each well as we had done during the screen and the first dose response tests

In this experiment, the DMSO negative control had a normalized value of 1±0.4 and the bFGF positive control had a value of 5.6±1.7. Both CPA and Budesonide were effective and produced proliferation levels much greater than that seen in the negative control. Without wishing to be bound by a theory, repeating these assays under the optimized experimental conditions the inventors have developed (300 cells/well plus a media change to refresh compounds after two days exposure) can improve both the variability of the assay as well as the proliferation response.

As with the hits from the first set of compounds screened, the inventors tested these compounds for ability to synergize with bFGF. Using inventors' standard culture conditions (10% horse serum in F-10), compounds were added to cells the day after plating, and refreshed daily until removed from the cultures as indicated. Cells were then grown in media without supplemented compounds until fixation on Day 4.

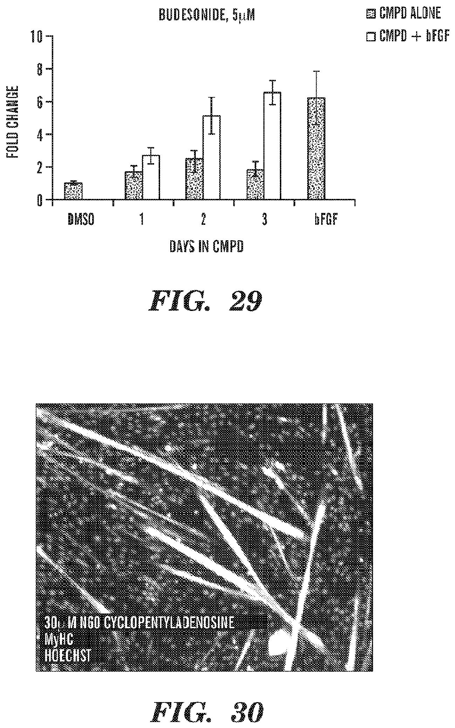

CPA (30µM) was seen to synergize with bFGF *in vitro.* The combination of CPA and bFGF showed a fifteen-fold increase in the cell number, as compared with approximately a six-fold increase with bFGF alone (**FIG. 28**). However, combination of Budesonide with bFGF provided no additional increase as compared to bFGF alone (**FIG. 29**).

The inventors also assessed effects these compounds were having on the identity of the satellite cells and their ability to differentiate in culture. For these experiments, 300 cells were plated in each well at Day 0 in our standard proliferation media

(10% horse serum in F-10). Compounds were added at Day 1 and SMPs were allowed to proliferate for three days. At Day 4, the media was switched to differentiation media (10% horse serum, 10%FBS, 0.5% chick embryo extract in high-glucose DMEM). Cultures were then incubated for three or four days under differentiation conditions and fixed on Day 7. They were then stained with Hoechst and anti-myosin heavy chain antibody. As can be seen in the **FIGS. 29 and 30****,** satellite cells exposed to the compound are able to fuse into myosin heavy chain-positive myotubes.

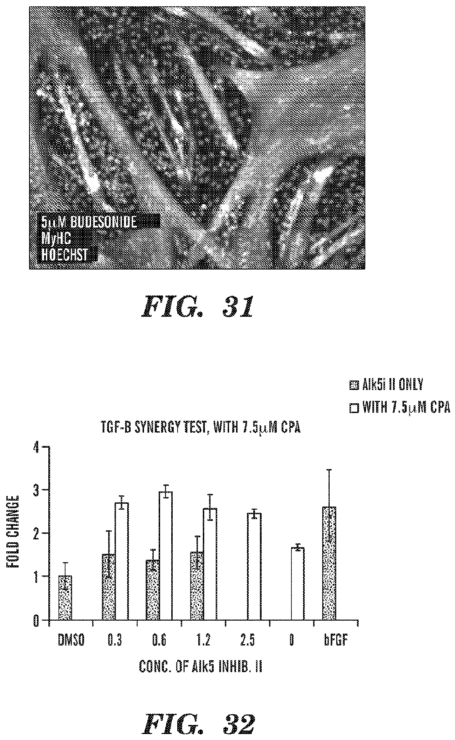

The inventors then tested whether these compounds can work additively with TGF-Beta inhibitors. A slight increase in proliferation was seen when the cells were treated with both CPA and Alk5 inhibitor II as compared to CPA and Alk5 inhibitor II alone (**FIG. 31**).

In this study, the inventors carried out a screen designed to identify compounds that are able to cause satellite cells to proliferate *in vitro.* The inventors discovered several compounds that can cause proliferation, both in the presence and absence of bFGF. The compounds gave rise to satellite cell populations that differentiate normally and carried normal markers for differentiation state. These results show that treatment with these compounds allow the satellite cell population to proliferate normally and contribute to muscle repair in disease states.

### Example 2

Satellite cells were harvested from CAG-B-actin-GFP mice, aged 2-4 months old via the FACS isolation method as outlined in Example 1. Briefly, all limb muscles and abdominal muscles were harvested from the animals and digested first in a 0.2% collagenase solution, then in a 0.0125% collagenase/0.05% dispase solution. The filtered solution was stained for cell surface markers, and put through FACS. Cells that were negative for Mac1, Sea1 and CD45 and positive for Beta-integrin 1 and CXCR4 were used for the screening assay. Cells were plated directly from the FACS machine at 50 cells/well, into 96-well plates coated with 10ug/mL laminin (4-6 hours at 37°C, then partially removed). Media for the screen was Ham's F-10 supplemented with 10% heat-inactivated horse serum, IX penicillin/streptomycin and IX L-glutamine. Basic FGF (bFGF) was added only to the positive control wells; all other wells received media only. The day of plating was called Day 0.

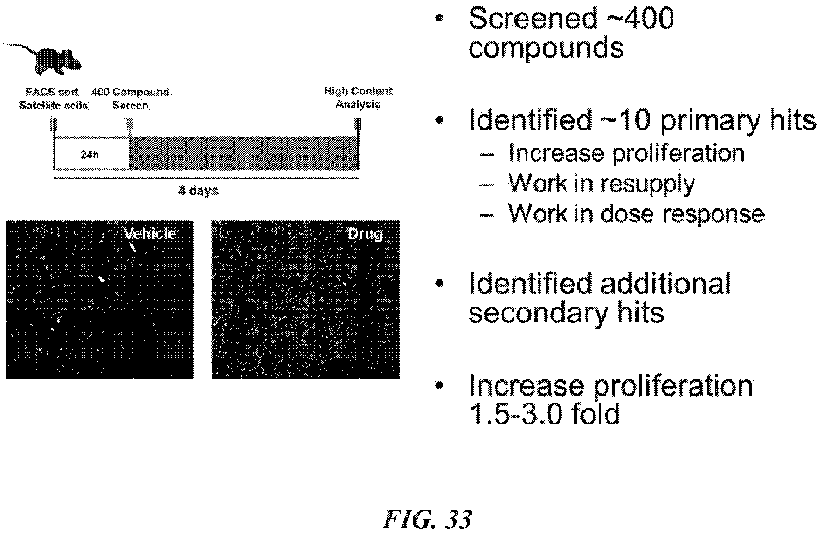

The day after plating (Day 1) the compounds were added, as illustrated in **FIG. 33****.** All compounds were dissolved in DMSO, and initially tested at 10uM, 1uM or 0.luM in duplicate. The negative control for each plate was the same concentration of DMSO (no compounds dissolved). Compounds were incubated with the cells until Day 4, with no media change. bFGF was spiked into positive control wells daily. On Day 4, plates were fixed in 4% paraformaldehyde and washed with phosphate buffered saline. Plates were imaged directly on the Opera Confocal imager, since the cells were readily visible from the CAG-EGFP-Beta-actin transgene. An Acapella script was used to count the number of cells in each well. Wells were scored for proliferation based on the cell counts (DMSO-treated wells usually had around 50 cells at the end of the assay, bFGF-treated wells usually had 150-250 cells at the end). Compounds were selected as hits if they had cell numbers greater than or equal to one (or, for the second session of screening, three) standard deviation(s) from the DMSO controls. Compounds thus selected were then tested in an initial dose curve; usually concentrations between 20nM-30uM commensurate with the concentration flagged as a hit. Compounds were deemed to be active if they were able to cause proliferation to cell numbers at least two standard deviations over the DMSO negative control. Any compounds that were found to be active in the dose curve were re-supplied from the original vendors. Re-supplied compounds were again tested in a dose curve for proliferation ability. Compounds still found to be active were then put in the queue for optimization and characterization.

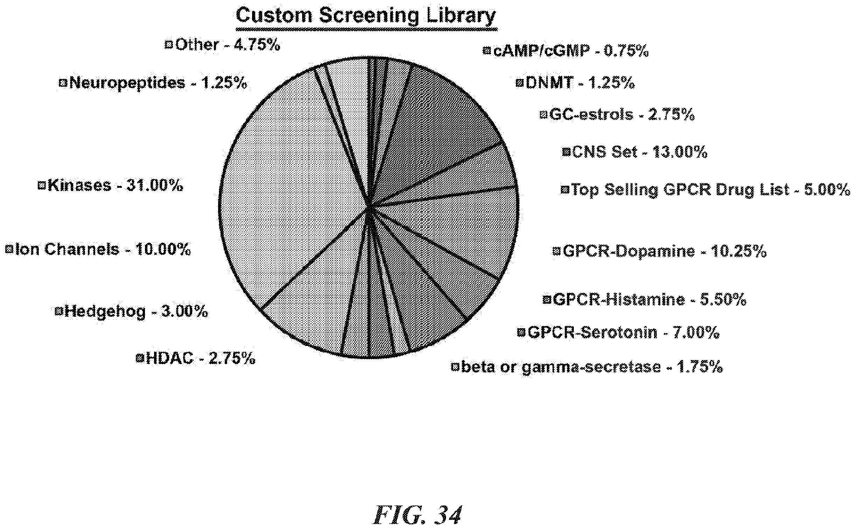

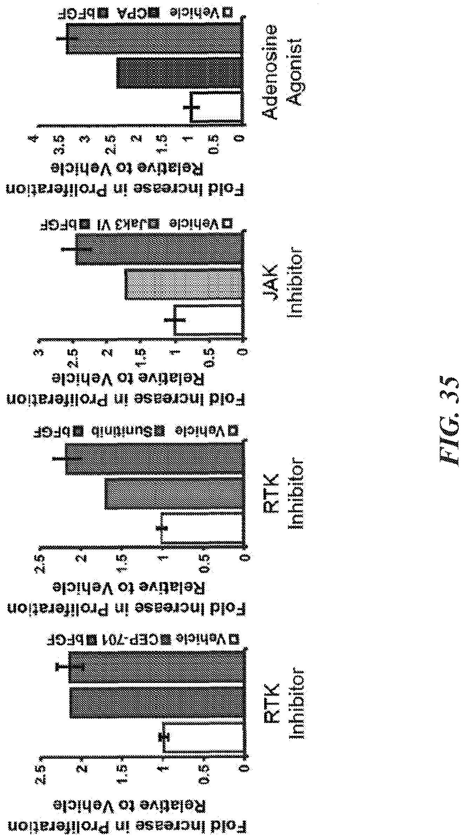

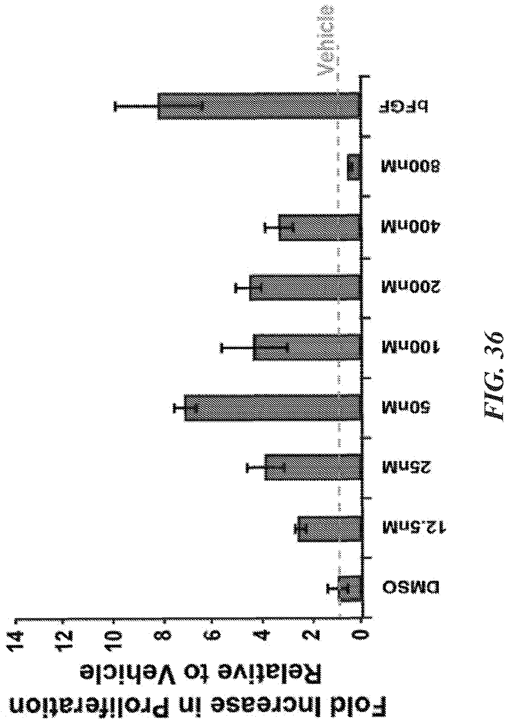

The inventors screened a set of approximately 400 compounds from the custom screening library illustrated in **FIG. 34****.** The inventors assayed these compounds at 1 µM, and counted as potential hits those compounds that scored greater than one standard deviation over the DMSO-treated negative control. Using these criteria, the inventors identified approximately 10 compounds from the set as hits that were capable of increasing *in vitro* satellite cell proliferation. The inventors then tested each of these compounds in a dose response assay to validate their activity. As illustrated in **FIG. 35****,** four of the compounds that were found to increase *in vitro* satellite cell proliferation were Lestaurtinib (CEP701), Sunitinib (SU11248), JAK3 inhibitor VI, and N6-cyclopentyladenosine (CPA). Lestaurtinib (CEP701) was identified as a top hit, was effective at nanomolar doses and had target overlap with several other hit compounds. Additionally, Lestaurtinib (CEP701) increased proliferation of aged satellite cells *in vitro* (**FIG. 36**), increased proliferation of human satellite cells and did not increase proliferation of fibroblasts.

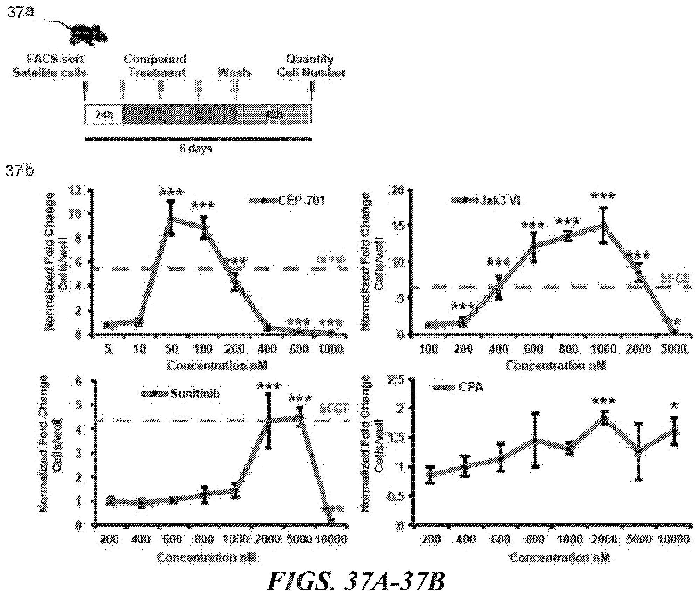

The inventors then tested each of these compounds in a dose response assay to validate their activity, as depicted in **FIG. 37A****.** Dose response curves for Lestaurtinib (CEP701), Sunitinib (SU11248), JAK3 inhibitor VI, and N6-cyclopentyladenosine (CPA) are shown in **FIG. 37B****.**

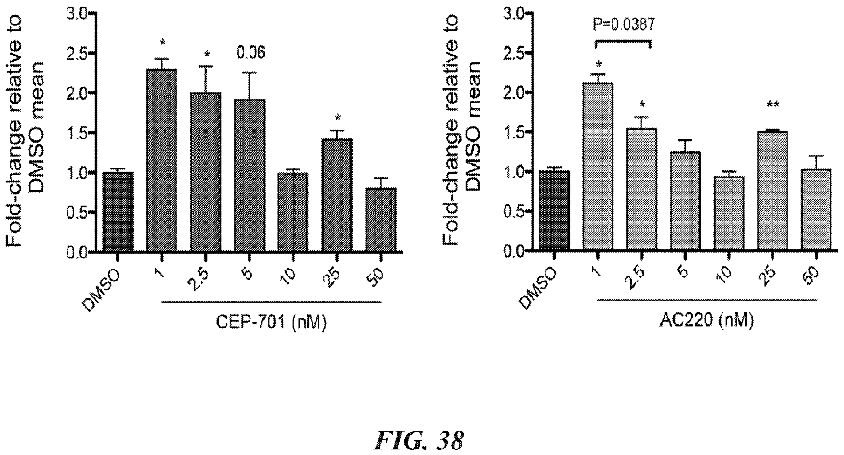

In addition to Lestaurtinib (CEP701), Quizartinib (AC220), a small molecule receptor tyrosine kinase inhibitor, also demonstrated an ability to expand human satellite cells at low doses. As illustrated in **FIG. 38****,** both CEP-701 and AC220 increased human satellite cells by more than 2-fold at a concentration of 1nM relative to the DMSO control.

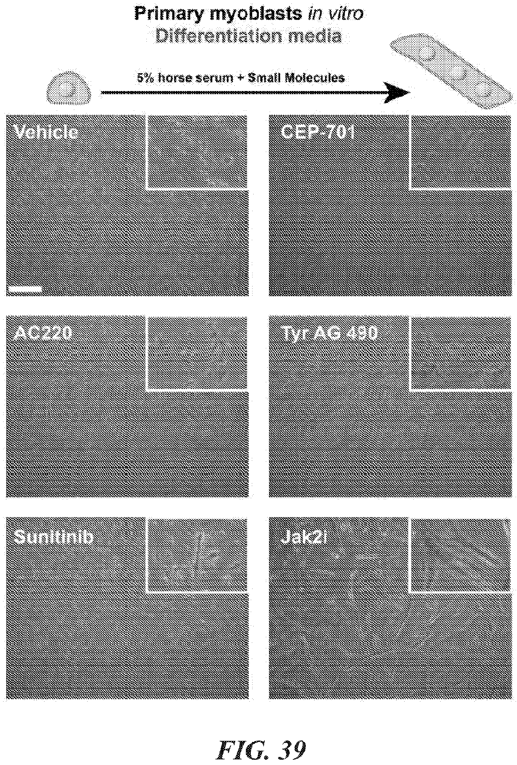

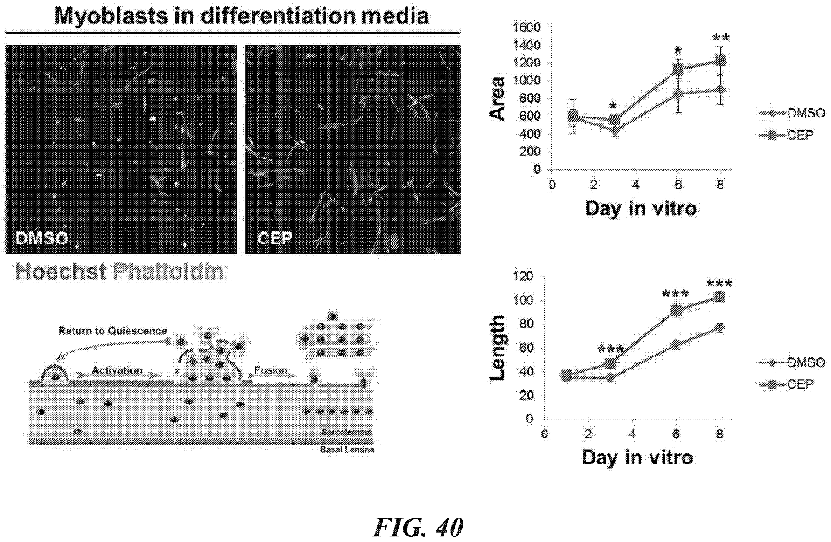

As illustrated in **FIG. 39****,** a differentiation media comprising 5% horse serum and those compounds identified as hits (e.g., CEP701, SU11248, JAK3 inhibitor VI, CPA and Tyr AG490) drive myoblast differentiation. Similarly, **FIG. 40** demonstrates that CEP701 enhances myoblast differentiation in differentiation media relative to the DMSO control, as evidenced by the observed increase in both myoblast area and length.

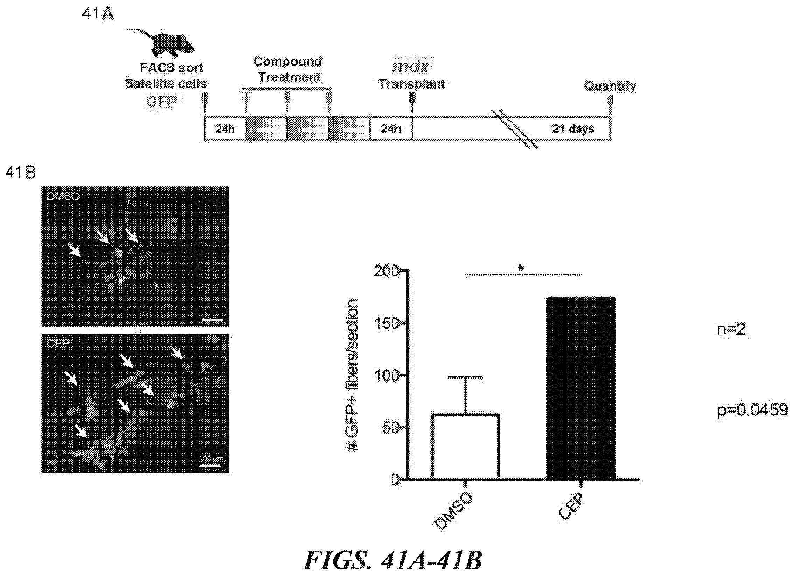

The present inventors next sought to determine whether CEP701-treated cells retain engraftability by performing the experimental protocol depicted in **FIG. 41A****,** by engrafting tubulin>GFP satellite cells into injured *mdx* muscle and administering CEP701 or a DMSO control. As illustrated in **FIG. 41B****,** CEP701 treated cells resulting in an increased number of GFP+ fibers per section, relative to the DMSO control.

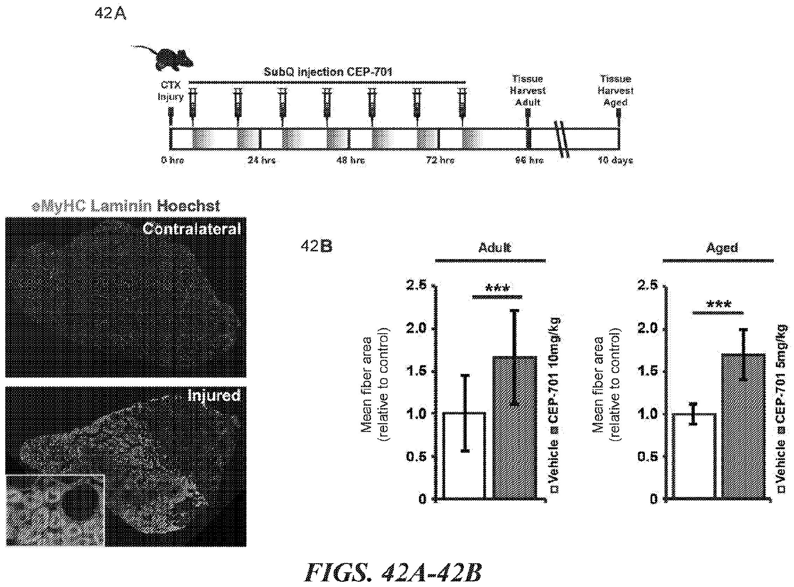

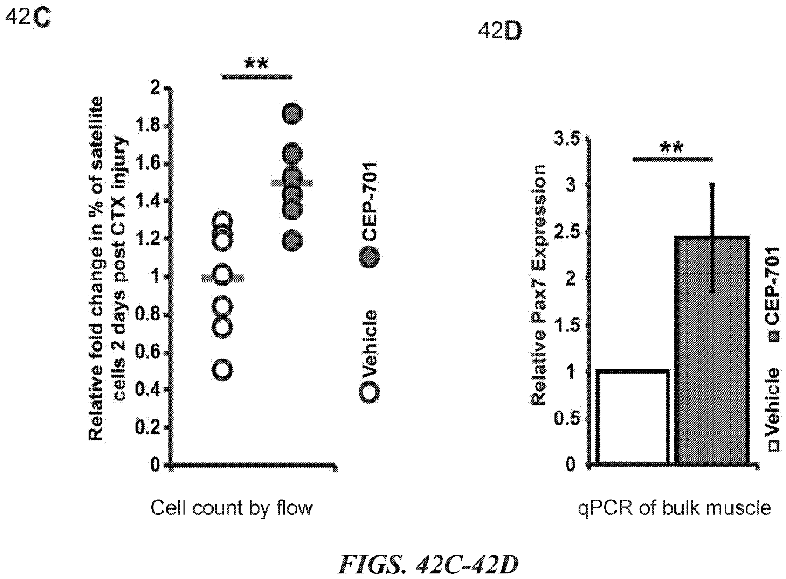

The present inventors next sought to determine whether CEP701 treatment increases regenerating fiber size and satellite cell number *in vivo*. CEP701 was administered subcutaneously to the mice post-CTX injury, followed by harvesting of the tissue, as depicted in **FIG. 42A****.** Regenerating muscle fibers are eMHC+. As illustrated in **FIGS. 42B-42D****,** treatment with CEP701 increased both regenerating fiber size and satellite cell number *in vivo* in both adult and aged mice.

The present inventors also performed an *in vitro* binding assay against active site fragments (KINOMEscan) in an effort to identify those multiple hit compounds that inhibit receptor tyrosine kinases (RTKs). As shown in **Table 4,** provided for information purposes below, CEP701, AC220 and sunitinib each inhibit multiple RTKs (numbers are Kd in nM; expression in primary myoblasts).

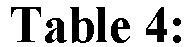

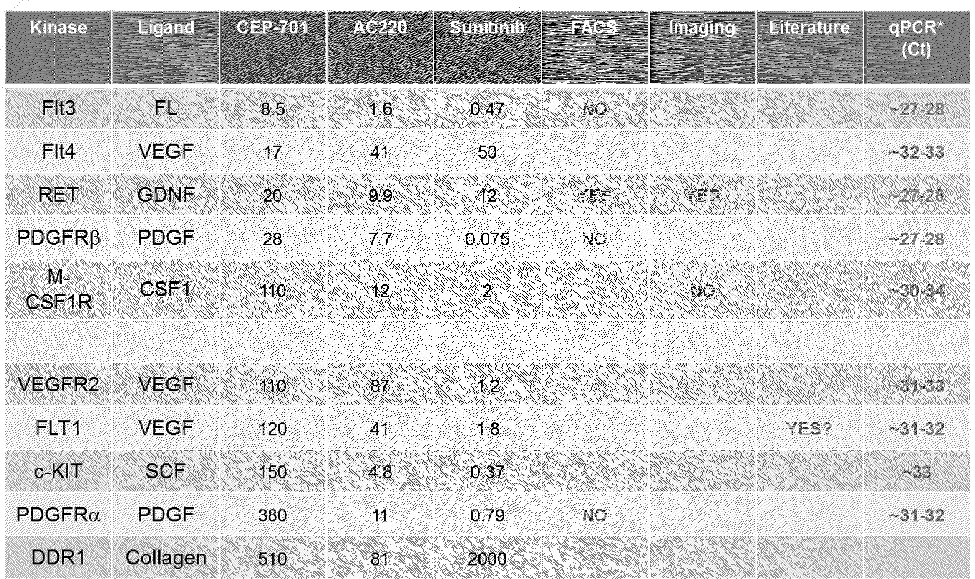

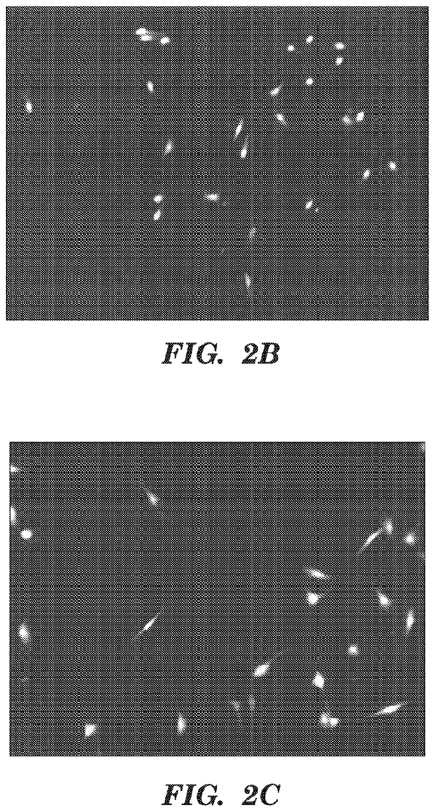

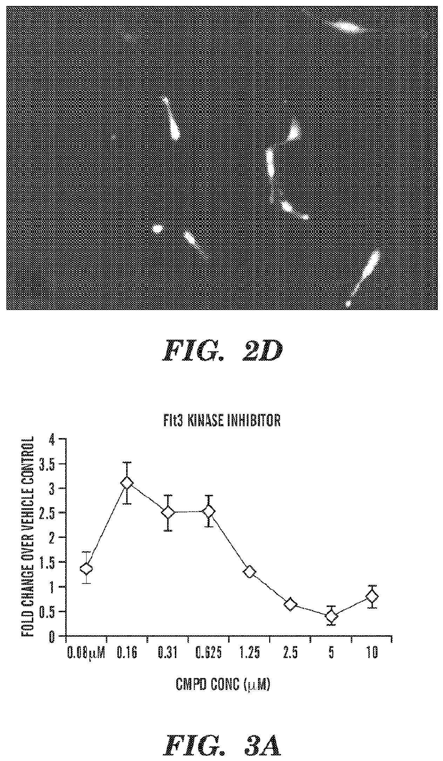

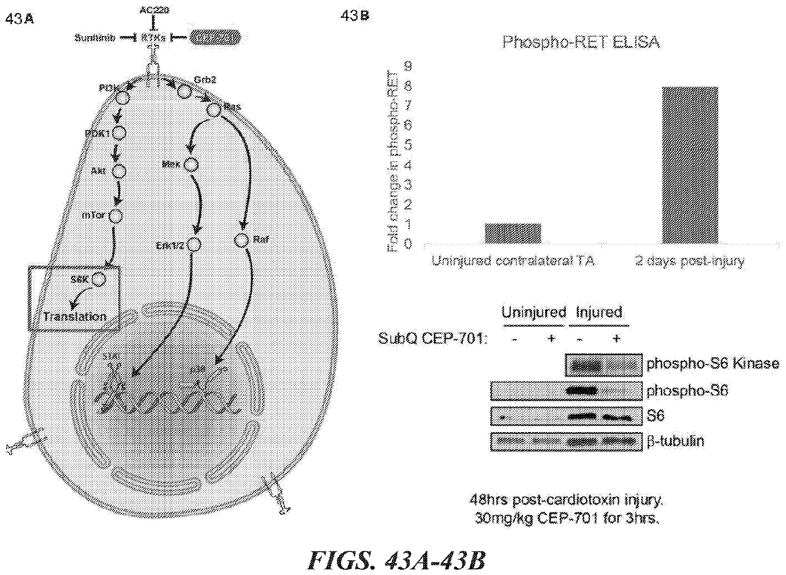

RET proto-oncogene is the receptor for the GDNF ligand family, which includes glial derived neurotrophic factor, neurturin, artemin and persephin, and is important for the development and function of several tissue types, including the nervous system. The RET proto-oncogene activates several downstream pathways, including JAK/STAT. As illustrated in **FIG. 43A****,** the multiple hit compounds CEP701, AC220 and sunitinib that are identified in **Table 4** inhibit the PDGFR family of RTKs. Phospho-RET levels are elevated in injured muscle, as illustrated in **FIG. 43B****,** which compares the fold change in phospho-RET in both uninjured contralateral tibialis anterior (TA) muscle to that observed 2 days post-cardiotoxin injury. As illustrated in **FIG. 43B****,** 2 days post-cardiotoxin injury, an approximately 8-fold increase in phospho-RET was observed by ELISA relative to uninjured contralateral TA muscle.

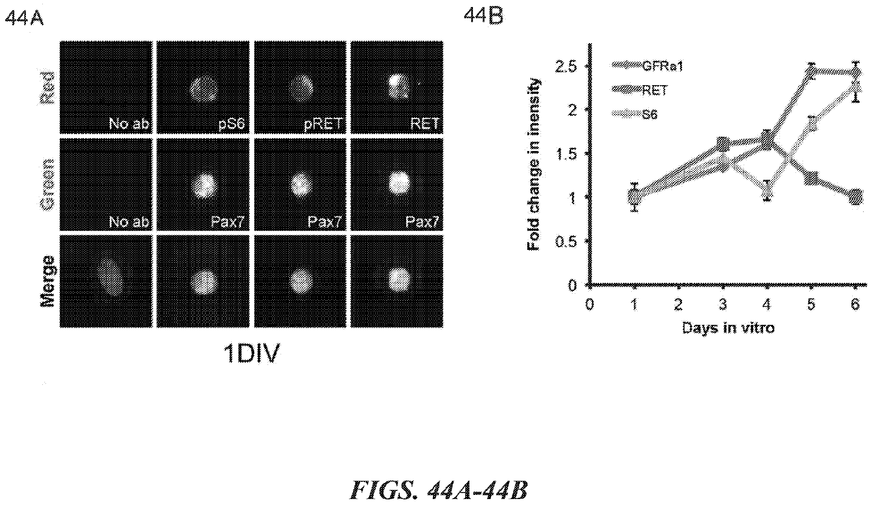

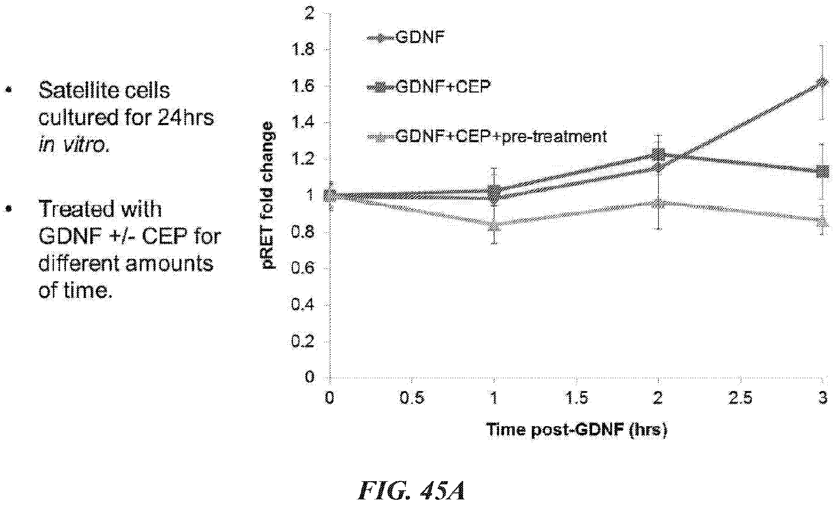

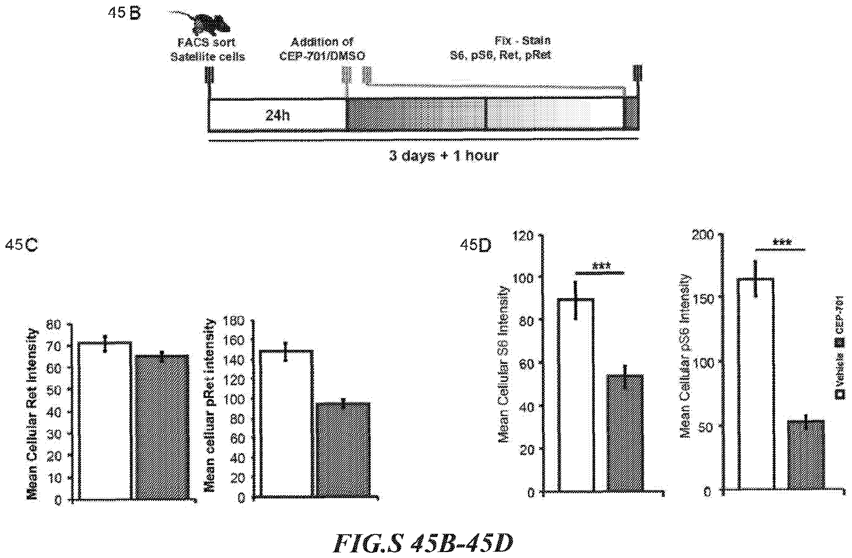

Satellite cells express RET *in vitro,* as shown in **FIGS. 44A-44B****.** As illustrated in **FIGS. 45A-45D****,** CEP701 treatment inhibits RET phosphorylation *in vitro.*

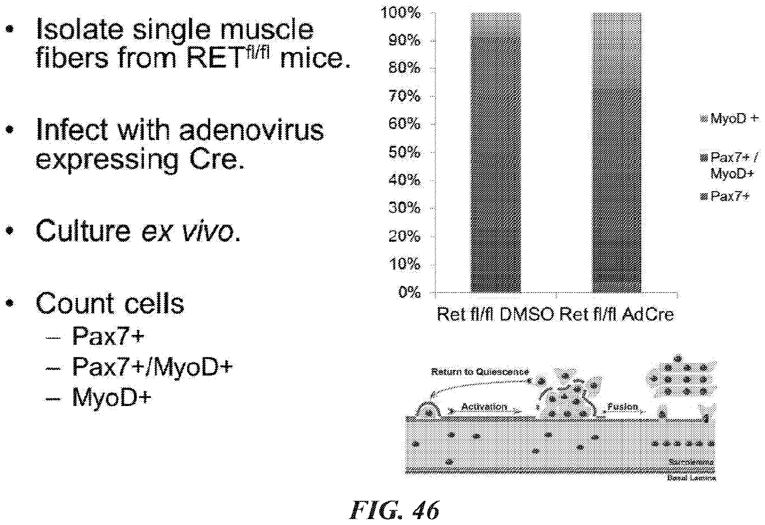

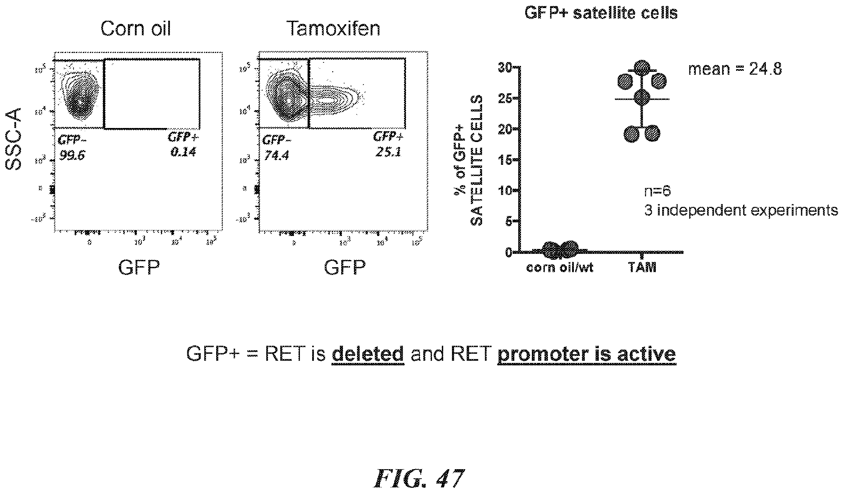

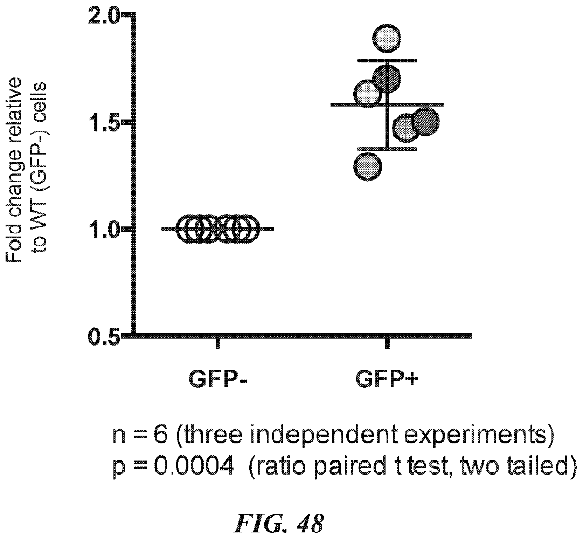

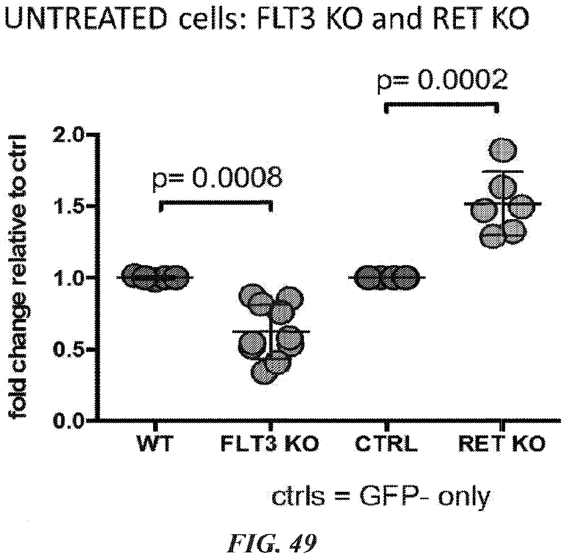

**FIG. 46** shows the results of a study evaluating the effects of the *in vitro* deletion of RET. Using a conditional RET mutant and reporter, the present inventors were able to determine that the RET promoter is active in at least 25% of satellite cells (**FIG. 47**) and that RET knockout cells proliferate better than wild-type cells *in vitro* (**FIG. 48**). **FIG. 49** demonstrates the fold change relative to control of untreated FLT3 and RET knockout cells.

### Example 3

This example includes a list of primary hits from a screen designed to identify compounds that promote satellite cell proliferation in vitro. The satellite cell is a skeletal muscle stem cell responsible for post-natal muscle growth and regeneration. Compounds that proliferate satellite cells in vitro have important implications for muscle regeneration because they have the potential to be equally effective in vivo, or to proliferate transplantable cells for cell replacement therapy. The four compound libraries tested, LINCS 1, 2, 3 and 4, were provided to us by the Sorger lab at Harvard Medical School. The Sorger lab is a member of the LINCS (Library of Integrated Network-Based Cellular Signatures) Consortium, which is an NIH initiative aiming to generate public data to further research of therapeutically relevant cellular pathways. LINCS 1, 2, and 4 contain kinase inhibitors, while LINCS 3 contains epigenetic modifiers. The results of the screen are shown in Table 5.

FIG. 50 identifies small molecules that promote satellite cell proliferation screen in this Example. (A) Chemical screen experimental schematic outlining FACS isolation and compound library treatment of satellite cells. (B) Representative dose response curves from four of the top ten compounds. Top ten compounds were chosen based on highest fold change of cell proliferation relative to vehicle controls. Proliferation was assessed via high content imaging using Hoechst 33342 as a cell marker. (C) Representative fluorescent images of FACS sorted satellite cells from Tg:Pax7-nGFP mice on 96w plates cultured for 4 days and treated with vehicle, compound or positive control (Jak3 inhibitor 6). Optimal treatment concentration for each compound was determined in dose response; 3uM for XMD8-92, 5uM for SB23906, 800nM for XMD11-50, and 400nM for Vorinostat. Hoechst 33342 was used as a cell marker. Scale bars denote 100um. (D) Fold change relative to vehicle control for several compounds that promote satellite cell expansion.

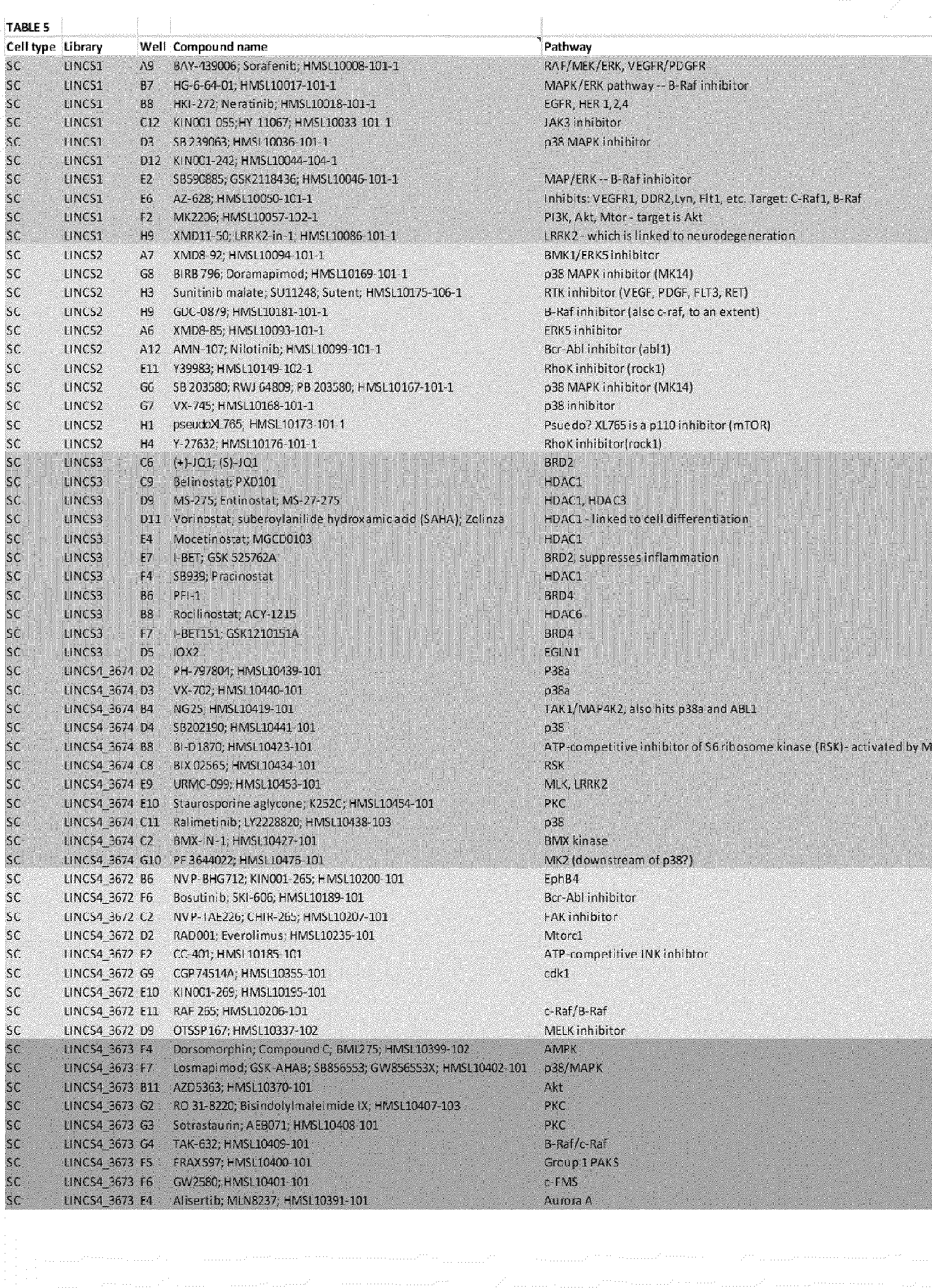

### Materials and Methods

### Satellite Cell Isolation and Chemical Screening.

Satellite cells were isolated from intact limb muscle and prepared for fluorescence activated cell sorting (FACS) as described previously (Rocheteau et al., 2012). FACS was performed at Harvard University at the Bauer Flow Cytometry Core Facility on a Beckman Coulter MoFlo Legacy (Beckman Coulter). Satellite cells were sorted into Eppendorf tubes, and plated onto 96 well plates by hand (Greiner). Plates were pre-coated at 37°C for 4 hours with 10ug/uL laminin diluted in PBS. Sorted cells were cultured in StemSpan SFEM II basal medium (Stem Cell Technologies) supplemented with 0.05ng/mL basic fibroblast growth factor (bFGF, Life Technologies). Wells for the positive control were treated with 600nM Jak3 inhibitor IV (EMD Millipore). For screening and dose response follow up, cells were plated at 150 cells/well.

### Compound Addition

The day cells were plated was considered Day 0. Compounds were added on Day 1. For screening and dose response follow up, cells were incubated without media change until Day 4 and plates were prepared for imaging. All compounds were suspended in Dimethylsulfoxide (DMSO, Sigma Aldrich); 0.1% DMSO was added as the negative control in all assays.

### References

Beharry, A.W., Sandesara, P.B., Roberts, B.M., Ferreira, L.F., Senf, S.M., and Judge, A.R. (2014). HDAC1 activates FoxO and is both sufficient and required for skeletal muscle atrophy. J Cell Sci 127, 1441-1453.
Bernet, J.D., Doles, J.D., Hall, J.K., Kelly Tanaka, K., Carter, T.A., and Olwin, B.B. (2014). p38 MAPK signaling underlies a cell-autonomous loss of stem cell self-renewal in skeletal muscle of aged mice. Nat Med 20, 265-271.
Charville, G.W., Cheung, T.H., Yoo, B., Santos, P.J., Lee, G.K., Shrager, J.B., and Rando, T.A. (2015). Ex Vivo Expansion and In Vivo Self-Renewal of Human Muscle Stem Cells. Stem Cell Reports 5, 621-632.
Palacios, D., Mozzetta, C., Consalvi, S., Caretti, G., Saccone, V., Proserpio, V., Marquez, V.E., Valente, S., Mai, A., Forcales, S.V., et al. (2010). TNF/p38alpha/polycomb signaling to Pax7 locus in satellite cells links inflammation to the epigenetic control of muscle regeneration. Cell Stem Cell 7, 455-469.
Price, F.D., von Maltzahn, J., Bentzinger, C.F., Dumont, N.A., Yin, H., Chang, N.C., Wilson, D.H., Frenette, J., and Rudnicki, M.A. (2014). Inhibition of JAK-STAT signaling stimulates adult satellite cell function. Nat Med.
Rocheteau, P., Gayraud-Morel, B., Siegl-Cachedenier, I., Blasco, M.A., and Tajbakhsh, S. (2012). A subpopulation of adult skeletal muscle stem cells retains all template DNA strands after cell division. Cell 148, 112-125.
Tierney, M.T., Aydogdu, T., Sala, D., Malecova, B., Gatto, S., Puri, P.L., Latella, L., and Sacco, A. (2014). STAT3 signaling controls satellite cell expansion and skeletal muscle repair. Nat Med 20, 1182-1186.
Troy, A., Cadwallader, A.B., Fedorov, Y., Tyner, K., Tanaka, K.K., and Olwin, B.B. (2012). Coordination of satellite cell activation and self-renewal by Par-complex-dependent asymmetric activation of p38alpha/beta MAPK. Cell Stem Cell 11, 541-553.

Although preferred embodiments have been depicted and described in detail herein, it will be apparent to those skilled in the relevant art that various modifications, additions, substitutions, and the like can be made without departing from the scope of the invention as defined in the claims which follow.

## Claims

1. A method of increasing satellite cell proliferation, the method comprising: contacting a satellite cell *in vitro* with a B-Raf inhibitor selected from the group consisting of BAY-439006 (i.e., Sorafenib; HMSL10008-101-1); HG-6-64-01 (i.e., HMSL10017-101-1); SB590885 (i.e., GSK2118436; HMSL10046-101-1); AZ-628 (i.e., HMSL10050-101-1); GDC-0879 (i.e., HMSL10181-101-1); RAF 265 (i.e., HMSL10206-101); TAK-632 (i.e., HMSL10409-101); and any combinations thereof.

2. The method of claim 1, wherein the compound is contacted with the satellite cell at a concentration of about 0.01nM to about 100µM.

3. The method of claim 1 or 2, wherein said contacting is for (a) at least 1 hour; or (b) one to seven days.

4. A B-Raf inhibitor selected from the group consisting of BAY-439006 (i.e., Sorafenib; HMSL10008-101-1); HG-6-64-01 (i.e., HMSL10017-101-1); SB590885 (i.e., GSK2118436; HMSL10046-101-1); AZ-628 (i.e., HMSL10050-101-1); GDC-0879 (i.e., HMSL10181-101-1); RAF 265 (i.e., HMSL10206-101); TAK-632 (i.e., HMSL10409-101); and any combinations thereof for use in muscle repair or regeneration in a subject which has a damaged muscle tissue.

5. The B-Raf inhibitor for use of claim 4, wherein the subject is a mammal; optionally wherein the subject is human.

6. The B-Raf inhibitor for use of claim 4 or 5, wherein the compound is co-administered with a therapeutic agent; optionally wherein the compound and the therapeutic agent are administered in the same formulation.

7. The B-Raf inhibitor for use of claims 4-6, wherein the compound is administered at a dosage of from 1 µg/kg to 150 mg/kg.

8. The B-Raf inhibitor for use of claims 4-7, wherein said administering is by injection, infusion, instillation, inhalation, or ingestion and/or said administering is once daily.

9. The B-Raf inhibitor for use of claims 4-8, further comprising diagnosing the subject for muscle damage or muscle atrophy/wasting before treating the subject for muscle repair or regeneration.

10. The B-Raf inhibitor for use of claim 4, wherein the damaged muscle tissue is the result of a physical injury or accident, disease, infection, over-use, loss of blood circulation, or muscle atrophy or wasting and/or wherein the damaged muscle tissue is dystrophic muscle or an ageing muscle and/or wherein the damaged muscle tissue is the result of muscle atrophy/wasting.

## Patentansprüche

1. Verfahren zur Steigerung der Proliferation von Satellitenzellen, wobei das Verfahren umfasst: das *In-vitro*-Inkontaktbringen einer Satellitenzelle mit einem B-Raf-Inhibitor, ausgewählt aus der Gruppe bestehend aus BAY-439006 (d. h. Sorafenib; HMSL.10008-101-1); HG-6-64-01 (d.h. HMSL10017-101-1); SB590885 (d.h. GSK2118436; HMSL10046-101-1); AZ-628 (d.h. HMSL10050-101-1); GDC-0879 (d.h. HMSL10181-101-1); RAF 265 (d.h. HMSL10206-101); TAK-632 (d. h. HMSL10409-101); und beliebigen Kombinationen davon.

2. Verfahren nach Anspruch 1, wobei die Verbindung in einer Konzentration von etwa 0,01 nM bis etwa 100 µM mit der Satellitenzelle in Kontakt gebracht wird.

3. Verfahren nach Anspruch 1 oder 2, wobei der Kontakt (a) mindestens 1 Stunde oder (b) ein bis sieben Tage andauert.

4. B-Raf-Inhibitor, ausgewählt aus der Gruppe bestehend aus BAY-439006 (d. h. Sorafenib; HMSL10008-101-1); HG-6-64-01 (d. h. HMSL10017-101-1); SB590885 (d.h. GSK2118436; HMSL10046-101-1); AZ-628 (d.h. HMSL10050-101-1); GDC-0879 (d.h. HMSL10181-101-1); RAF 265 (d. h. HMSL10206-101); TAK-632 (d. h. HMSL10409-101); sowie beliebigen Kombinationen davon zur Verwendung bei der Muskelreparatur oder - regeneration bei einem Patienten mit geschädigtem Muskelgewebe.

5. B-Raf-Inhibitor zur Verwendung nach Anspruch 4, worin es sich bei dem Patienten um ein Säugetier handelt; wahlweise worin es sich bei dem Patienten um einen Menschen handelt.

6. B-Raf-Inhibitor zur Verwendung nach Anspruch 4 oder 5, wobei die Verbindung zusammen mit einem Therapeutikum verabreicht wird; wahlweise wobei die Verbindung und das Therapeutikum in derselben Formulierung verabreicht werden.

7. B-Raf-Inhibitor zur Verwendung nach den Ansprüchen 4-6, wobei die Verbindung in einer Dosierung von 1 µg/kg bis 150 mg/kg verabreicht wird.

8. B-Raf-Inhibitor zur Verwendung nach den Ansprüchen 4-7, wobei die Verabreichung durch Injektion, Infusion, Instillation, Inhalation oder Einnahme erfolgt und/oder die Verabreichung einmal täglich erfolgt.

9. B-Raf-Inhibitor zur Verwendung nach den Ansprüchen 4-8, wobei die Verwendung ferner das Diagnostizieren eines Muskelschadens oder einer Muskelatrophie/Muskelschwund beim Patienten vor der Behandlung des Patienten zur Muskelreparatur oder -regeneration umfasst.

10. B-Raf-Inhibitor zur Verwendung nach Anspruch 4, worin das geschädigte Muskelgewebe das Ergebnis einer körperlichen Verletzung oder eines Unfalls, einer Erkrankung, einer Infektion, einer Überbeanspruchung, eines Durchblutungsverlusts oder einer Muskelatrophie bzw. eines Muskelschwunds ist und/oder worin das geschädigte Muskelgewebe dystrophisches Muskelgewebe oder alternder Muskel ist und/oder worin das geschädigte Muskelgewebe das Ergebnis einer Muskelatrophie bzw. eines Muskelschwunds ist.

## Revendications

1. Procédé d'augmentation de la prolifération de cellules satellites, le procédé comprenant : la mise en contact d'une cellule satellite *in vitro* avec un inhibiteur de B-Raf choisi dans le groupe consistant en BAY-439006 (c'est-à-dire, Sorafénib ; HMSL10008-101-1) ; HG-6-64-01 (c'est-à-dire, HMSL10017-101-1) ; SB590885 (c'est-à-dire, GSK2118436 ; HMSL10046-101-1) ; AZ-628 (c'est-à-dire, HMSL10050-101-1) ; GDC-0879 (c'est-à-dire, HMSL10181-101-1) ; RAF 265 (c'est-à-dire, HMSL10206-101) ; TAK-632 (c'est-à-dire, HMSL10409-101) ; et toute combinaison de ceux-ci.

2. Procédé selon la revendication 1, dans lequel le composé est mis en contact avec la cellule satellite à une concentration d'environ 0,01 nM à environ 100 µM.

3. Procédé selon la revendication 1 ou 2, dans lequel ladite mise en contact est pendant (a) au moins 1 heure ; ou (b) un à sept jours.

4. Inhibiteur de B-Raf choisi dans le groupe consistant en BAY-439006 (c'est-à-dire, Sorafénib ; HMSL10008-101-1) ; HG-6-64-01 (c'est-à-dire, HMSL10017-101-1) ; SB590885 (c'est-à-dire, GSK2118436 ; HMSL10046-101-1) ; AZ-628 (c'est-à-dire, HMSL10050-101-1) ; GDC-0879 (c'est-à-dire HMSL10181-101-1) ; RAF 265 (c'est-à-dire HMSL10206-101) ; TAK-632 (c'est-à-dire HMSL10409-101) ; et toute combinaison de ceux-ci pour une utilisation dans la réparation ou la régénération musculaire chez un sujet qui présente un tissu musculaire lésé.

5. Inhibiteur de B-Raf pour une utilisation selon la revendication 4, dans lequel le sujet est un mammifère ; facultativement dans lequel le sujet est humain.

6. Inhibiteur de B-Raf pour une utilisation selon la revendication 4 ou 5, dans lequel le composé est co-administré avec un agent thérapeutique ; facultativement dans lequel le composé et l'agent thérapeutique sont administrés dans la même formulation.

7. Inhibiteur de B-Raf pour une utilisation selon les revendications 4 à 6, dans lequel le composé est administré à une dose de 1 µg/kg à 150 mg/kg.

8. Inhibiteur de B-Raf pour une utilisation selon les revendications 4 à 7, dans lequel ladite administration est par injection, perfusion, instillation, inhalation ou ingestion et/ou ladite administration est une fois par jour.

9. Inhibiteur de B-Raf pour une utilisation selon les revendications 4 à 8, comprenant en outre le diagnostic chez le sujet de lésions musculaires ou d'atrophie/fonte musculaire avant de traiter le sujet pour réparation ou régénération musculaire.

10. Inhibiteur de B-Raf pour une utilisation selon la revendication 4, dans lequel le tissu musculaire lésé est le résultat d'une blessure physique ou d'un accident, d'une maladie, d'une infection, d'une surutilisation, d'une perte de circulation sanguine ou d'une atrophie ou fonte musculaire et/ou dans lequel le tissu musculaire lésé est un muscle dystrophique ou un muscle vieillissant et/ou dans lequel le tissu musculaire lésé est le résultat d'une atrophie/fonte musculaire.
